# EUROPEAN PATENT APPLICATION

(11) **EP 1 903 045 A1**
(43) Date of publication of application: **26.03.2008**
(21) Application number: 06756776.8
(22) Date of filing: 24.05.2006
(51) Int. Cl.: C07D 487/04, A61K 31/519, A61P 5/04, A61P 25/22, A61P 25/24, A61P 43/00, C07D 231/14

(54) **PYRAZOLOPYRIMIDINE DERIVATIVE**

(30) Priority: 27.05.2005 JP 2005155310
(71) Applicant: Mitsubishi Tanabe Pharma Corporation, Chuo-ku Osaka-shi (JP)
(72) Inventor: KASHIWAGI, Toshihiko, Chuo-ku, Osaka-shi Osaka 541-8505 (JP); TAKAMURO, Iwao, Chuo-ku, Osaka-shi Osaka 541-8505 (JP); WATANABE, Yumi, Chuo-ku, Osaka-shi Osaka 541-8505 (JP); YATO, Michihisa, Chuo-ku, Osaka-shi Osaka 541-8505 (JP)
(74) Representative: HOFFMANN EITLE
(86) International application number: PCT/JP2006/310828
(87) International publication number: WO 2006/126718

(57) **Abstract**

A pyrazolopyrimidine derivative of the formula [I] 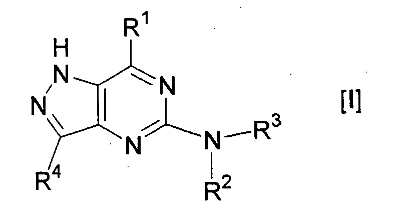 wherein R¹ is an optionally substituted aromatic ring group, an optionally substituted lower alkyl group or an optionally substituted amino group; R² is an optionally substituted aromatic ring group; R³ is an optionally substituted lower alkyl group; and R⁴ is a hydrogen atom, a lower alkyl group, a halogen atom, a nitro group or an amino group;
or a pharmaceutically acceptable salt thereof is useful as an antagonist of CRF receptor.

## Description

### BACKGROUND OF THE INVENTION

The present invention relates to a pyrazolopyrimidine derivative, which is useful as a receptor antagonist of CRF (Corticotropin Releasing Factor), a pharmaceutical acceptable salt and a synthetic intermediate thereof.

### SUMMARY OF THE INVENTION

It is expected that a receptor antagonist of CRF is developed as a preventive and/or therapeutic agent for depression, anxiety disorder, irritable bowel syndrome (IBS) and the like, and a compound of pyrazolo[1,5-a]pyrimidine is disclosed in WO 2005/026126, for example. However, none of pyrazolo[4,3-d]pyrimidine is disclosed in the PCT gazzete.

### DISCLOSURE OF THE INVENTION

The present invention provides a novel pyrazolopyrimidine derivative having an excellent activity as a CRF receptor antagonist. The inventors have extensively studied to find novel pyrazolopyrimidine derivatives having a CRF-receptor antagonistic activity and finally completed the invention. That is, the present invention provides the followings;
1. a pyrazolopyrimidine derivative of the generic formula [I], 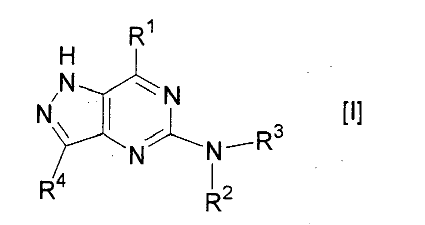 wherein R¹ is an optionally substituted aromatic ring group, an optionally substituted lower alkyl group or an optionally substituted amino group; R² is an optionally substituted aromatic ring group; R³ is an optionally substituted lower alkyl group; and R⁴ is a hydrogen atom, a lower alkyl group, a halogen atom, a nitro group or an amino group;
   or a pharmaceutically acceptable salt thereof.
2. a compound of 1. above, wherein R¹ is an optionally substituted aromatic ring group; R² is an optionally substituted aromatic ring group; R³ is a lower alkyl group; and R⁴ is a hydrogen atom.
3. a compound of 1. above, wherein R¹ is an aromatic ring group optionally substituted with 1-5 group(s) selected from a lower alkoxy group optionally substituted with 1-5 halogen atom(s), a halogen atom, an optionally substituted amino group and an optionally substituted alkyl group; R² is an aromatic ring group substituted with 1-5 group(s) selected from a halogen atom, a lower alkoxy group, a lower alkyl group optionally substituted with 1-5 halogen atom(s),an optionally substituted carbamoyl group and an optionally substituted amino group; R³ is a lower alkyl group; and R⁴ is a hydrogen atom.
4. a compound of 1. above, wherein R¹ is an aromatic ring group optionally substituted with 1-5 group(s) selected from a lower alkoxy group optionally substituted with 1-5 halogen atom(s) and a halogen atom; R² is an aromatic ring group substituted with 1-5 group(s) selected from a halogen atom, a lower alkoxy group and a lower alkyl group optionally substituted with 1-5 halogen atom(s); R³ is a lower alkyl group; and R⁴ is a hydrogen atom.
5. a compound of 4. above, wherein R¹ is a phenyl group or quinolyl group optionally substituted with 1-5 group(s) selected from a lower alkoxy group optionally substituted with 1-5 halogen atom(s) and a halogen atom; and R² is a phenyl group substituted with 1-5 group(s) selected from a halogen atom, a lower alkoxy group and a lower alkyl group optionally substituted with 1-5 halogen atom(s).
6. N-[2-chloro-4-(trifluoromethyl)phenyl]-N-ethyl-7-(2-methoxy-6-fluorophenyl)-1H-pyrazolo[4,3-d]pyrimidine-5-amine,
   N-[2-chloro-4-(trifluoromethyl)phenyl]-N-ethyl-7-quinoline-8-yl-1H-pyrazolo[4,3-d]pyrimidine-5-amine,
   N-(2-chloro-4-methoxyphenyl)-N-ethyl-7-(2-methoxyphenyl)-1H-pyrazolo[4,3-d]pyrimidine-5-amine,
   N-[2-chloro-4-(trifluoromethyl)phenyl]-N-ethyl-7-[2-(trifluoromethoxy)phenyl]-1H-pyrazolo[4,3-d]pyrimidine-5-amine,
   N-[2-chloro-4-(trifluoromethyl)phenyl]-N-ethyl-7-(2-ethoxy-6-fluorophenyl)-1H-pyrazolo[4,3-d]pyrimidine-5-amine,
   N-[2-chloro-4-(trifluoromethyl)phenyl]-7-[2-(difluoromethoxy)phenyl]-N-ethyl-1H-pyrazolo[4,3-d]pyrimidine-5-amine,
   N-[2-chloro-4-(trifluoromethyl)phenyl]-7-(2,3-difluolo-6-methoxyphenyl)-N-ethyl-1H-pyrazolo[4,3-d]pyrimidine-5-amine,
   N-[2-chloro-4-(trifluoromethyl)phenyl]-7-[2-(cyclopropylmethoxy)phenyl]-N-ethyl-1H-pyrazolo[4,3-d]pyrimidine-5-amine,or
   N-(2-chloro-4-methoxyphenyl)-N-ethyl-7-(2-fluoro-6-methoxyphenyl)-1H-pyrazolo[4,3-d]pyrimidine-5-amine,
   or a pharmaceutically acceptable salt thereof.
7. a compound of the formula [II], [III], [V], [VII], [X], [XI], [XII] or the generic formula [XIII], 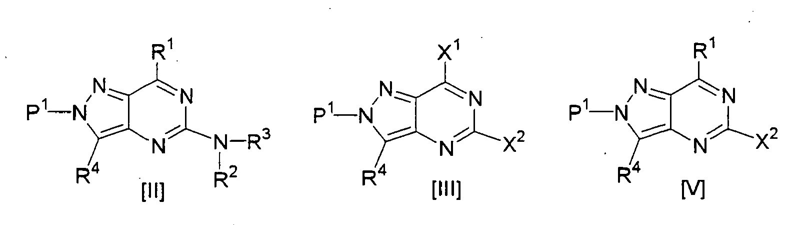 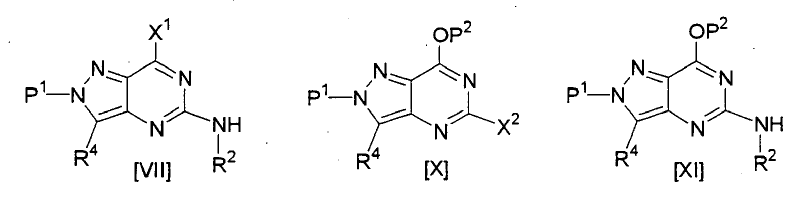 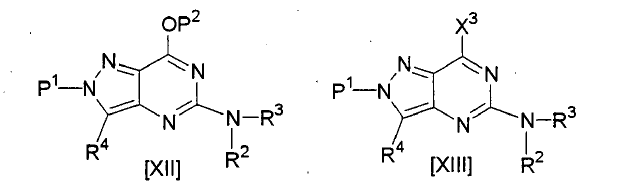 wherein R¹ is an optionally substituted aromatic ring group, an optionally substituted lower alkyl group or an optionally substituted amino group; R² is an optionally substituted aromatic ring group; R³ is an optionally substituted lower alkyl group; and R⁴ is a hydrogen atom, a lower alkyl group, a halogen atom, a nitro group or an amino group; P¹ and P² are protecting groups and X¹, X² and X³ are leaving groups;
   or a salt thereof.
8. methyl 1-(4-methoxybenzyl)-4-nitro-1H-pyrazole-3-carboxylate,
   1-(4-methoxybenzyl)-4-nitro-1H-pyrazole-3-carboxamide,
   4-amino-1-(4-methoxybenzyl)-1H-pyrazole-3-carboxamide,
   2-(4-methoxybenzyl)-2H-pyrazolo[4,3-d]pyrimidine-5,7-(4H,6H)-dione,
   5,7-dichloro-2-(4-methoxybenzyl)-2H-pyrazolo[4,3-d]pyrimidine, or
   or a salt thereof.

Compounds of the present invention are explained below in detail.

A 5- to 12-membered aromatic ring is preferred and a heteroatom such as nitrogen atom, oxygen atom, sulfur atom and the like may be included as an aromatic ring of the present invention. The concrete examples include a phenyl group, a naphthyl group, a pyridyl group, a pyrimidyl group, a quinolyl group, an isoquinolyl group, a pyrazolyl group and an oxazolyl group etc.

Examples of a substituent group of the aromatic ring include a lower alkyl group, a lower alkoxy group, a halogen atom, a dihalogeno lower alkyl group, a trihalogeno lower alkyl group, a dihalogeno lower alkoxy group, a trihalogeno lower alkoxy group, a nitro group, an amino group, a lower alkyl amino group, a di(lower alkyl)amino group, a di(lower alkyl)amino lower alkyl group, a hydroxyl group, a lower alkanoyl group, a lower alkanoylamino group, a morpholinyl group, a morpholinyl-substituted lower alkyl group, a lower alkoxy carbonyl group, a lower alkoxy-lower alkyl group,a lower alkoxy lower alkoxy group, a lower alkyleneoxy group, a lower alkoxycarbonyl lower alkyl group, a cycloalkyl group, a cycloalkyl-substituted lower alkyl group, a cycloalkyl-substituted lower alkoxy group, a phenyl group, a lower alkylthio group, a lower alkylsulfonyl group, a lower alkylsulfinyl group, a lower alkyl sulfonyl amino group, a pyrrolidinyl group, a piperidyl group, a cyano group, an oxetane-substituted lower alkoxy group, an oxolane-substituted oxy group, a lower alkylthio lower alkoxy group, a hydroxyl lower alkyl group, a carbamoyl group, a di(lower alkyl)carbamoyl group, a pyrrolidinylcarbonyl group, a cycloalkyl-substituted carbonyl group, a cycloalkylcarbamoyl group, a phenyl-substituted lower alkyl group, a lower alkanoylamino lower alkyl group, a di(lower alkyl)amino lower alkoxy group, a hexa-halogeno hydroxyl lower alkyl group, a cyano lower alkoxy group, a carbamoyl lower alkoxy group, a phenyl lower alkoxy group and the like.

Examples of substituents of the lower alkyl group include a pyridyl group, a lower alkoxycarbonyl group, a halogen atom and the like.

Examples of substituents of the amino group include an alkyl group, an alkoxy-substituted alkyl group, a trihalogeno alkyl group, an aryl-substituted alkyl group, a heteroaryl-substituted alkyl group and an aromatic ring group, and further a substituent which may construct an optionally substituted heterocyclo group combined with a nitrogen atom. Examples of the substituents on said heterocyclo group include a lower alkoxy group, a lower alkoxy-substituted lower alkyl group, a hydroxy-substituted lower alkyl group and the like.

In the present invention, it may be substituted with not only one but two or more substituents.

A concrete example of the compound [I] of the present invention includes a compound in which R¹ is (1) an aromatic ring group optionally substituted with one or more group(s) selected from a lower alkyl group, a lower alkoxy group, a halogen atom, a dihalogeno lower alkyl group, a trihalogeno lower alkyl group, a dihalogeno lower alkoxy group, a trihalogeno lower alkoxy group, a nitro group, an amino group, a lower alkylamino group, a di(lower alkyl)amino group, a di(lower alkyl)amino lower alkyl group, a hydroxy group, a lower alkanoyl group, a lower alkanoylamino group, a morpholinyl group, a morpholinyl-substituted lower alkyl group, a lower alkoxycarbonyl, a lower alkoxy lower alkyl group, a lower alkoxy lower alkoxy group, a lower alkyleneoxy group, a lower alkoxycarbonyl lower alkyl group, a cycloalkyl group, a cycloalkyl-substituted lower alkyl group, a cycloalkyl-substituted lower alkoxy group, a phenyl group, a lower alkylthio group, a lower alkylsulfonyl group, a lower alkylsulfinyl group, a lower alkylsulfonylamino group, a pyrrolidinyl group, a piperidinyl group, a cyano group, an oxetane-substituted lower alkoxy group, an oxolane-substituted oxy group, a lower alkylthio lower alkoxy group, a hydroxyl lower alkoxy group, a carbamoyl group, a di(lower alkyl) carbamoyl group, a pyrrolidinylcarbonyl group, a cycloalkyl-substituted carbonyl group, a cycloalkyl carbamoyl group, a phenyl lower alkyl group, a lower alkanoylamino lower alkyl group, a di(lower alkyl)amino lower alkoxy group, a hexahalogeno-hydroxy lower alkyl group, a cyano lower alkoxy group, a carbamoyl lower alkoxy group and a phenyl lower alkoxy group, (2) a lower alkyl group optionally substituted with one or more group(s) selected from a pyridyl group, a lower alkoxycarbonyl group and a halogen atom (3)an amino group optionally substituted with one or more group(s) selected from an alkyl group, an alkoxy-substituted alkyl group, a trihalogeno alkyl group, an aryl-substituted alkyl group, a heteroaryl-substituted alkyl group and an aromatic ring group or (4) a heterocyclo group optionally substituted with one or more group(s) selected from a lower alkoxy group, a lower alkoxy-substituted lower alkyl group and a hydroxyl lower alkyl group; R² is an aromatic ring group optionally substituted with one or more group(s) selected from a lower alkyl group, a lower alkoxy group, a halogen atom, a dihalogeno lower alkyl group, a trihalogeno lower alkyl group, a dihalogeno lower alkoxy group, a trihalogeno lower alkoxy group, a nitro group, an amino group, a lower alkylamino group, a di(lower alkyl)amino group, a di(lower alkyl)amino lower alkyl group, a hydroxy group, a lower alkanoyl group, a lower alkanoylamino group, a morpholinyl group, a morpholinyl-substituted lower alkyl group, a (lower alkoxy)carbonyl, a lower alkoxy lower alkyl group, a lower alkoxy lower alkoxy group, a lower alkyleneoxy group, a lower alkoxycarbonyl lower alkyl group, a cycloalkyl group, a cycloalkyl-substituted lower alkyl, a cycloalkyl-substituted lower alkoxy group, a phenyl group, a lower alkylthio group, a lower alkylsulfonyl group, a lower alkylsulfinyl group, a lower alkylsulfonylamino group, a pyrrolidinyl group, a piperidinyl group, a cyano group, an oxetane-substituted lower alkoxy group, an oxolane-substituted oxy group, a lower alkylthio lower alkoxy group, a hydroxyl lower alkoxy group, a carbamoyl group, a di(lower alkyl) carbamoyl group, a pyrrolidinylcarbonyl group, a cycloalkyl-substituted carbonyl group, a cycloalkyl carbamoyl group, a phenyl lower alkyl group, a lower alkanoylamino lower alkyl group, a di(lower alkyl)amino lower alkoxy group, a hexahalogeno-hydroxy lower alkyl group, a cyano lower alkoxy group, a carbamoyl lower alkoxy group and a phenyl lower alkoxy group; R³ is a lower alkyl group optionally substituted with one or more group(s) selected from a pyridyl group, a lower alkoxycarbonyl group and a halogen atom; and R⁴ is a hydrogen atom, a lower alkyl group, a halogen atom, a nitro group or an amino group.

Examples of preferred compounds in the present invention include a compound wherein R¹ is an optionally substituted aromatic ring group or an optionally substituted lower alkyl group, R² is an optionally substituted aromatic ring group, R³ is a lower alkyl group optionally substituted with a halogen atom and R⁴ is a hydrogen atom.

Examples of particularly preferred compounds in the present invention include the following;
N-[2-Chloro-4-(trifluoromethyl)phenyl]-N-ethyl-7-(2-methoxy-6-fluorophenyl)-1H-pyrazolo[4,3-d]pyrimidine-5-amine,
N-[2-Chloro-4-(trifluoromethyl)phenyl]-N-ethyl-7-quinoline-8-yl-1H-pyrazolo[4,3-d]pyrimidine-5-amine,
N-(2-Chloro-4-methoxyphenyl)-N-ethyl-7-(2-methoxyphenyl)-1H-pyrazolo[4,3-d]pyrimidine-5-amine,
N-[2-Chloro-4-(triflnoromethyl)phenyl]-N-ethyl-7-[2-(trifluoromethoxy)phenyl]-1H-pyrazolo[4,3-d]pyrimidine-5-amine,
N-[2-Chloro-4-(trifluoromethyl)phenyl]-N-ethyl-7-(2-ethoxy-6-fluorophenyl)-1H-pyrazolo[4,3-d]pyrimidine-5-amine,
N-[2-Chloro-4-(trifluoromethyl)phenyl]-7-[2-(difluoromethoxy)phenyl]-N-ethyl-1H-pyrazolo[4,3-d]pyrimidine-5-amine,
N-[2-Chloro-4-(trifluoromethyl)phenyl]-7-(2,3-difluoro-6-methoxyphenyl)-N-ethyl-1H-pyrazolo[4,3-d]pyrimidine-5-amine,
N-[2-Chloro-4-(trifluoromethyl)phenyl]-7-[2-(cyclopropylmethoxy)phenyl]-N-ethyl-1H-pyrazolo[4,3-d]pyrimidine-5-amine,
N-(2-Chloro-4-methoxyphenyl)-N-ethyl-7-(2-fluoro-6-methoxyphenyl)-1H-pyrazolo[4,3-d]pyrimidine-5-amine,
N-(2-Chloro-4-isopropylphenyl)-N-ethyl-7-(2-methoxyphenyl)-1H-pyrazolo[4,3-d]pyrimidine-5-amine,
N²,N⁵-Diethyl-N⁵-[7- (2-methoxyphenyl)-1H-pyrazolo[4,3-d]pyrimidine-5-yl]-N²,N⁵-dimethylpyridine-2,5-diamine
N-[2-Chloro-4-(trifluoromethyl)phenyl]-N-ethyl-7-quinoline-yl-1H-pyrazolo[4,3-d]pyrimidine-5-amine,
N-[2-Chloro-4-(trifluoromethyl)phenyl]-N-ethyl-7-(1-naphthyl)-1H-pyrazolo[4,3-d]pyrimidine-5-amine,
N-[2-Chloro-4-(trifluoromethyl)phenyl]-N-ethyl-7-[2-(trifluoromethyl)phenyl]-1H-pyrazolo[4,3-d]pyrimidine-5-amine,
N-[2-Chloro-4-(trifluoromethyl)phenyl]-7-(2-ethoxyphenyl)-N-ethyl-1H-pyrazolo[4,3-d]pyrimidine-5-amine,
7-(2-Chlorophenyl)-N-[2-chloro-4-(trifluoromethyl)phenyl]-N-ethyl-1H-pyrazolo[4,3-d]pyrimidine-5-amine,
7-(5-Chloro-2-methoxyphenyl)-N-[2-chloro-4-(trifluoromethyl)phenyl]-N-ethyl-1H-pyrazolo[4,3-d]pyrimidine-5-amine,
N-[2-Chloro-4-(trifluoromethyl)phenyl]-7-(2,6-dimethoxyphenyl)-N-ethyl-1H-pyrazolo[4,3-d]pyrimidine-5-amine,
N-[2-Chloro-4-(trifluoromethyl)phenyl]-N-ethyl-7-(3-fluoro-2-methoxyphenyl)-1H-pyrazolo[4,3-d]pyrimidine-5-amine,
N-[2-Chloro-4-(trifluoromethyl)phenyl]-N-ethyl-7-(2-morpholine-4-ylphenyl)-1H-pyrazolo[4,3-d]pyrimidine-5-amine,
N-[2-Chloro-4-(trifluoromethyl)phenyl]-N-7-(5-fluoro-2-methoxyphenyl)-1H-pyrazolo[4,3-d]pyrimidine-5-amine,
N-[2-Chloro-4-(trifluoromethyl)phenyl]-7-(2-ethoxy-1-naphthyl)-N-ethyl-1H-pyrazolo[4,3-d]pyrimidine-5-amine,
N-[2-Chloro-4-(trifluoromethyl)phenyl]-7-(2-ethoxy-5-fluorophenyl)-N-ethyl-1H-pyrazolo[4,3-d]pyrimidine-5-amine,
N-Ethyl-(4-isopropyl-2,6-dimethoxyphenyl)-7-(2-methoxyphenyl)-1H-pyrazolo[4,3-d]pyrimidine-5-amine,
N-(2-Chloro-4,6-dimethoxyphenyl)-N-ethyl-7-(2-methoxyphenyl)-1H-pyrazolo[4,3-d]pyrimidine-5-amine,
N-(2-chloro-4-(trifluoromethyl)phenyl)-7-[2-(dimethylamino)phenyl]-N-ethyl-1H-pyrazolo[4,3-d]pyrimidine-5-amine,
N-[2-Chloro-4-(trifluoromethyl)phenyl]-N-ethyl-7-(2-propoxyphenyl)-1H-pyrazolo[4,3-d]pyrimidine-5-amine,
N-[2-Chloro-4-(trifluoromethyl)phenyl]-N-ethyl-7-[2-methoxy-6-(methylthio)phenyl]-1H-pyrazolo[4,3-d]pyrimidine-5-amine,
N-[2-Chloro-4-(trifluoromethyl)phenyl]-N-ethyl-7-[2-(methoxymethyl)phenyl]-1H-pyrazolo[4,3-d]pyrimidine-5-amine,
N-Ethyl-7-(2-fluoro-6-methoxyphenyl)-N-[2-methyl-4-(trifluoromethyl)phenyl]-1H-pyrazolo[4,3-d]pyrimidine-5-amine,
N-(2-Chloro-4-methylphenyl)-N-ethyl-7-(2-fluoro-6-methoxyphenyl)-1H-pyrazolo[4,3-d]pyrimidine-5-amine,
N-[2-Chloro-4-(trifluoromethyl)phenyl]-N-ethyl-7-(2-methylpheny)-1H-pyrazolo[4,3-d]pyrimidine-5-amine,
N-[2-Chloro-4-(trifluoromethyl)phenyl]-7-(3,5-difluoro-2-methoxyphenyl)-N-ethyl-1H-pyrazolo[4,3-d]pyrimidine-5-amine,
N-[2-Chloro-4-(trifluoromethyl)phenyl]-N-ethyl-7-(2-isopropoxyphenyl)-1H-pyrazolo[4,3-d]pyrimidine-5-amine,
N-[2-Chloro-4-(trifluoromethyl)phenyl]-7-(2,6-difluorophenyl)-N-ethyl-1H-pyrazolo[4,3-d]pyrimidine-5-amine,
N-[2-Chloro-4-(trifluoro)phenyl]-7-[2-difluoromethoxy]-N-ethyl-1H-pyrazolo[4,3-d]pyrimidine-5-amine,
N-[2-Chloro-4-(ethoxymethyl)-5-methoxyphenyl]-N-ethyl-7-(2-fluoro-6-methoxyphenyl)-1H-pyrazolo[4,3-d]pyrimidine-5-amine,
N-[2-Chloro-4-(trifluoromethyl)phenyl]-7-(2,3-difluoro-6-methoxyphenyl)-N-ethyl-1H-pyrazolo[4,3-d]pyrimidine-5-amine,
N-[2-Chloro-4-(trifluoromethyl)phenyl]-N-ethyl-7-(2-pyrrolidine-1-ylphenyl)-1H-pyrazolo[4,3-d]pyrimidine-5-amine,
N-[2-Chloro-4-(trifluoromethyl)phenyl]-7-[2-(cyclopropylmethoxy)phenyl]-N-ethyl-1H-pyrazolo[4,3-d]pyrimidine-5-amine,
N-Ethyl-7-(2-fluoro-6-methoxyphenyl)-N-[2-methoxy-4-(trifluoromethyl)phenyl]-1H-pyrazolo[4,3-d]pyrimidine-5-amine,
N¹-Ethyl-N¹-[7-(2-fluoro-6-methoxyphenyl)-1H-pyrazolo[4,3-d]pyrimidine-5-yl]-N²,N²-dimethyl-4-(trifluoromethyl)benzene-1,2-diamine
N-(2-Chloro-4-nitro-phenyl)-N-ethyl-7-(2-fluoro-6-methoxyphenyl)-1H-pyrazolo[4,3-d]pyrimidine-5-amine,
N-[2-Chloro-4-(difluoromethyl)phenyl]-N-ethyl-7-(2-fluoro-6-methoxyphenyl)-1H-pyrazolo[4,3-d]pyrimidine-5-amine,
N-[2-Chloro-4-(trifluoromethyl)phenyl]-N-ethyl-7-[2-fluoro-6-(2-methoxyethoxy)phenyl]-1H-pyrazolo[4,3-d]pyrimidine-5-amine,
N-[2-Chloro-4-(trifluoromethyl)phenyl]-7-(2-ethoxy-3-fluorophenyl)-N-ethyl-1H-pyrazolo[4,3-d]pyrimidine-5-amine,
N-[2-Chloro-4-(trifluoromethyl)phenyl]-N-ethyl-7-(3-fluoro-2-propoxyphenyl)-1H-pyrazolo[4,3-d]pyrimidine-5-amine,
3-Chloro-4-[N-ethyl-7-(2-fluoro-6-methoxyphenyl)-1H-pyrazolo[4,3-d]pyrimidine-5-amino]benzonitrile
N-[2-Chloro-4-(trifluoromethyl)phenyl]-7-[2-(cyclopropylmethoxy)-6-fluorophenyl]-N-ethyl-1H-pyrazolo[4,3-d]pyrimidine-5-amine
N-[2-Chloro-4-(trifluoromethyl)phenyl]-N-ethyl-7-[2-fluoro-6-(tetrahydrofuran-3-yloxy)phenyl]-1H-pyrazolo[4,3-d]pyrimidine-5-amine,
N-[2-Chloro-4-(trifluoromethoxy)phenyl]-N-ethyl-7-(2-fluoro-6-methoxyphenyl)-1H-pyrazolo[4,3-d]pyrimidine-5-amine,
N-(2-Chloro-5-fluoro-4-methoxyphenyl)-N-ethyl-7-(2-fluoro-6-methoxyphenyl)-1H-pyrazolo[4,3-d]pyrimidine-5-amine,
2-Chloro-N¹-ethyl-5-fluoro-N¹-[7-(2-fluoro-6-methoxyphenyl)]-1H-pyrazolo[4,3-d]pyrimidine-5-yl]-N⁴,N⁴-dimethylbenzene-1,4-diamine
N-(2-Chloro-4-ethoxy-5-fluorophenyl)-N-ethyl-7-(2-fluoro-6-methoxyphenyl)-1H-pyrazolo[4,3-d]pyrimidine-5-amine,
N-(2-Chloro-4-methoxyphenyl)-N-ethyl-7-(2-fluoro-6-methoxyphenyl)-1H-pyrazolo[4,3-d]pyrimidine-5-amine,
N-Ethyl-N-(2-fluoro-4-methoxyphenyl)-7-(2-fluoro-6-methoxyphenyl)-1H-pyrazolo[4,3-d]pyrimidine-5-amine,
N-[2,4-Dichloro-5-(2-methoxyethoxy)phenyl]-N-ethyl-7-(2-fluoro-6-methoxyphenyl)-1H-pyrazolo[4,3-d]pyrimidine-5-amine,
N-[2-Chloro-4-(trifluoromethyl)phenyl]-N-ethyl-7-(2-fluoro-6-isopropoxyphenyl)-1H-pyrazolo[4,3-d]pyrimidine-5-amine,
N-[2-chloro-4-(ethoxymethyl)-5-fluorophenyl]-N-ethyl-7-(2-fluoro-6-methoxyphenyl)-1H-pyrazolo[4,3-d]pyrimidine-5-amine,
N-(4-Chloro-2-methoxyphenyl)-N-ethyl-7-(2-fluoro-6-methoxyphenyl)-1H-pyrazolo[4,3-d]pyrimidine-5-amine,
N-[2-Chloro-4-(trifluoromethyl)phenyl]-N-ethyl-7-[2-fluoro-6-(2,2,2-fluoroethoxy)phenyl]-1H-pyrazolo[4,3-d]pyrimidine-5-amine,
[2-[5-[N-[2-chloro-4-(trifluoromethyl)phenyl]-(ethyl)amino]-1H-pyrazolo[4,3-d]pyrimidine-7-yl]-3-fluorophenoxy]acetonitrile
N-Ethyl-7-(2-fluoro-6-methoxyphenyl)-N-(2,4,6-trichlorophenyl) -1H-pyrazolo[4,3-d]pyrimidine-5-amine,
N-(2,4-Dichlorophenyl)-N-ethyl-7-(2-fluoro-6-methoxyphenyl)-1H-pyrazolo[4,3-d]pyrimidine-5-amine,
N-[2-Chloro-4-(trifluoromethyl)phenyl]-7-(6-ethoxy-2,3-difluorophenyl)-N-ethyl-1H-pyrazolo[4,3-d]pyrimidine-5-amine,
N-[2-Chloro-4-(trifluoromethyl)phenyl]-7-[6-(cyclopropylmethoxy)-2,3-difluorophenyl]-N-ethyl-1H-pyrazolo[4,3-d]pyrimidine-5-amine,
N-(2,4-Dichloro-6-methylphenyl)-N-ethyl-7-(2-fluoro-6-methoxyphenyl)-1H-pyrazolo[4,3-d]pyrimidine-5-amine,
N-[2-Chloro-4-(trifluoromethyl)phenyl]-7-[2-(cyclobutyloxy)phenyl]-N-ethyl-1H-pyrazolo[4,3-d]pyrimidine-5-amine,
N-[2-Chloro-4-(trifluoromethyl)phenyl]-N-ethyl-7-[2-(tetrahydrofuran-3-yloxy)phenyl]-1H-pyrazolo[4,3-d]pyrimidine-5-amine,
N-[2-Chloro-4-(trifluoromethyl)phenyl]-7-[2-(cyclopentyloxy)phenyl]-N-ethyl-1H-pyrazolo[4,3-d]pyrimidine-5-amine,
N-[2-chloro-4-(trifluoromethyl)phenyl]-N-ethyl-7-[2-(1-ethylpropoxy)phenyl]-1H-pyrazolo[4,3-d]pyrimidine-5-amine,
N-[2-Chloro-4-(trifluoromethyl)phenyl]-N-ethyl-7-(2-fluoro-6-propoxyphenyl)-1H-pyrazolo[4,3-d]pyrimidine-5-amine,
N-[2-chloro-4-(trifluoromethyl)phenyl]-N-ethyl-7-[2-(2,2,2-trifluoroethoxy)phenyl]-1H-pyrazolo[4,3-d]pyrimidine-5-amine,
(2-{5-[N-[2-Chloro-4-(trifluoromethyl)phenyl](ethyl)amino]-1H-pyrazolo[4,3-d]pyrimidine-7-yl}phenoxy)acetonitrile
N-[2,6-Dichloro-4-(trifluoromethoxy)phenyl]-N-ethyl-7-(2-fluoro-6-methoxyphenyl)-1H-pyrazolo[4,3-d]pyrimidine-5-amine,
3,5-Dichloro-4-[N-ethyl[7-(2-fluoro-6-methoxyphenyl)-1H-pyrazolo[4,3-d]pyrimidine-5-yl]amino]benzonitrile,
N-[2-Chloro-4-(trifluoromethyl)phenyl]-7-[2-(cyclohexyloxy)phenyl]-N-ethyl-1H-pyrazolo[4,3-d]pyrimidine-5-amine,and
N-[2-chloro-4-(trifluoromethyl)phenyl]-N-ethyl-7-[2-(tetrahydro-2H-pyrane-4-yloxy)phenyl]-1H-pyrazolo[4,3-d]pyrimidine-5-amine.

In addition, examples of the lower alkyl group or the lower alkoxy group include a straight or branched carbon chain having one to six carbon atom(s), and especially a straight or branched carbon chain having one to four carbon atom(s) is preferrable.

Examples of the alkyl group or alkoxy group include a straight or branched carbon chain having one to ten carbon atom(s).

Examples of the cycloalkyl group include a group having three to eight carbon atoms, and especially a group having three to six carbon atoms is preferable.

Examples of the halogen atom include a fluorine atom, a chlorine atom, a bromine atom and an iodine atom, and especially a fluorine atom, a chlorine atom and a bromine atom are preferable.

Examples of the aryl group include a phenyl group, a naphthyl group and the like, and examples of the heteroaryl group include a pyridyl group, a furyl group, a thienyl group and the like. Examples of the heterocyclo group include an imidazolyl group, a piperidyl group, a pyrrolyl group, a quinolyl group, a tetrahydroquinolyl group and the like.

The compound [I] of the present invention can be clinically used either in the free form or in the form of a pharmaceutically acceptable salt thereof. Examples of the pharmaceutically acceptable salt of the compound [I] include for example, an inorganic acid salt such as hydrochloride, sulfate, phosphate or hydrobromide; and an organic acid salt such as acetate, fumarate, oxalate, citrate, methanesulfonate, benzenesulfonate, tosylate or maleate. Besides, when the compound [I] of the present invention has a carboxyl group(s) and the like in its molecule, examples of the pharmaceutically acceptable salt include salts with a base such as alkaline metal (e.g., a sodium salt, a potassium salt) or alkaline earth metal (e.g., a calcium salt).

In addition, the compound [I] of the present invention include isolated isomers such as a geometrical isomer, a tautomer or an optical isomer and a mixture thereof.

Further, the present invention includes intramolecular salts, hydrates, a pharmaceutically acceptable solvates, or crystal polymorphism of the compound [I]. Obviously, the compounds of the present invention should be construed to include all of the compounds shown by the generic formula [I] or pharmaceutically acceptable salt thereof, and not to be limited to the compound illustrated in the following examples.

The objective compound [I] or its pharmaceutically acceptable salt of the present invention shows an antagonistic activity against CRF receptors.

Accordingly, the objective compound [I] or its pharmaceutically acceptable salt of the present invention may be used as an agent for the treatment or prevention of depression, anxiety disorder, irritable bowel syndrome(IBS) and the like. Moreover, the objective compound [I] of the present invention is less toxic and has the feature as a safe drug.

The objective compound [I] of the present invention or a pharmaceutically acceptable salt thereof can be administrated either orally or parenterally, and can be formulated into a conventional pharmaceutical preparation such as tablets, granules, capsules, powders, injections or inhalants.

The dose of the compound [I] of the present invention or a pharmaceutically acceptable salt thereof may vary in accordance with the administration routes, and the ages, weights and conditions of the patients. For example, when administered in an injection preparation, it is usually in the range of about 0.01 to 100 mg/kg/day, preferably in the range of about 0.01 to 10 mg/kg/day. When administered in an oral preparation, it is usually in the range of about 0.1 to 100 mg/kg/day, preferably in the range of 0.1 to 10 mg/kg/day.

According to the present invention, the compound [I] or pharmaceutically acceptable salt thereof can be prepared by the following methods but should not be construed to be limited thereto.

### Method for preparing the compound [I]

The compound [I] or a pharmaceutically acceptable salt thereof can be prepared by deprotection of P¹ in a compound of the generic formula [II] 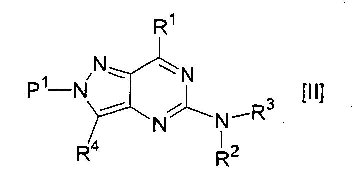 wherein P¹ is a protecting group and the other symbols have the same meaning as above, and conversion of the product to a pharmaceutically acceptable salt thereof, if necessary.

### Method for preparing the compound [II]

The compound [II] can be prepared by either Method A or Method B below, but should not be construed to be limited thereto.

### [Method A]

The compound [II] can be prepared by reacting a compound of the generic formula [III] 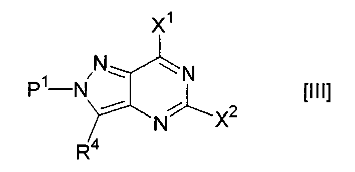 wherein X¹ and X² are leaving groups and the other symbols have the same meaning as above,
with a compound of the generic formula [IV]

R¹-H [IV]

wherein the symbol has the same meaning as above, or a reactive derivative thereof, to give a compound of the generic formula [V] 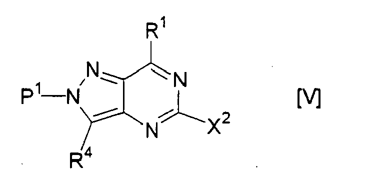 wherein the symbols have the same meaning as above, next, reacting the compound [V] with a compound of the generic formula [VI]

R²-NH₂ [VI]

wherein the symbol has the same meaning as above, to give a compound of the generic formula [VII] 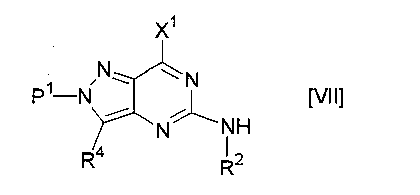 wherein the symbols have the same meaning as above, and further reacting the compound [VII] with a compound of the generic formula [VIII]

R³- L² [VIII]

wherein L² is a leaving group and the other symbols have the same meaning as above.

### [Method B]

The compound [II] can be prepared by reacting a compound of the generic formula [IX]

P²O-H [IX]

wherein P² is a protecting group and the other symbol has the same meaning as above, with the compound [III] to give a compound of the generic formula [X] 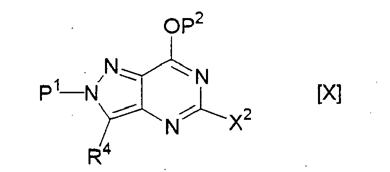 wherein the symbols have the same meaning as above, next, reacting the compound [X] with the compound [VI] to give a compound of the generic formula [XI] 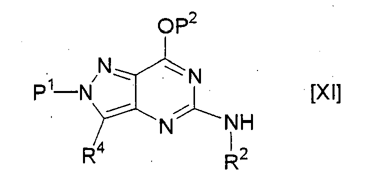 wherein the symbols have the same meaning as above, next, reacting the compound [XI] with the compound [VIII] to give a compound of the generic formula [XII] 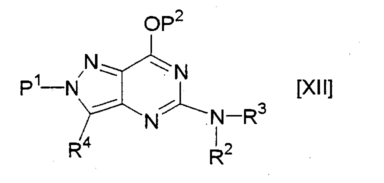 wherein the symbols have the same meaning as above, next converting the OH group to a leaving group after deprotection of the P² group to give a compound of the generic formula [XIII] 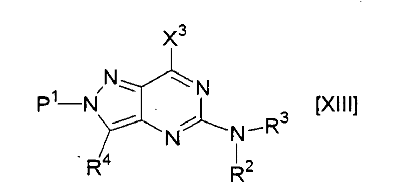 wherein X³ is a leaving group and the other symbols have the same meaning as above,
and finally reacting the compound [XIII] with the compound [IV] or a reactive derivative thereof.

### Reaction of the preparation of the compound [I]

A conventional amino-protecting group may be used as a protecting group of P¹ and examples of P¹ include a 4-methoxybenzyl group, a benzyloxycarbonyl group, a 4-methoxybenzyloxycarbonyl group, a t-butoxycarbonyl group, an acetyl group, a benzoyl group, a benzyl group, a tosyl group and the like.

Deprotection of the protecting group P¹ in the compound [II] can be carried out by the conventional method such as acid or base treatment in an appropriate solvent or without a solvent or a catalytic reduction for example. As an example of the acid, an inorganic acid such as hydrochloric acid, sulfuric acid or an organic acid such as acetic acid, trifluoroacetic acid, methanesulfonic acid, p-toluenesulfonic acid can be preferably used. As an example of the base, an inorganic base(for example, alkali metal hydride such as sodium hydride; alkali metal carbonate such as sodium carbonate, potassium carbonate; alkali metal amide such as sodium amide, lithium amide; alkali metal alkoxide such as sodium methoxide; alkali metal such as sodium; alkali metal hydroxide such as sodium hydroxide, potassium hydroxide etc.) can be preferably used.

The catalytic reduction can be carried out by using palladium carbon, palladium hydroxide carbon, platinum oxide, Raney nickel and the like under hydrogen atmosphere.

Examples of the solvent include any solvent which does not disturb the reaction, such methanol, ethanol, isopropyl alcohol, propyl alcohol, 1,4-dioxane, methylenechloride, chloroform, dichloroethane, ether, tetrahydrofuran, ethyl acetate, toluene and a mixture thereof. Besides, acids or bases described above can be used as a solvent. The reaction can be carried out preferably at a temperature from -78°C to a boiling point of the solvent.

### [Reaction of Method A]

Examples of leaving groups shown by X¹ and X² include a halogen atom, a lower alkylthio group, a lower alkylsulfinyl group, a lower alkylsulfonyl group optionally substituted with one to three halogen atom(s), an arylsulfonyloxy group and a lower alkylsulfonyloxy group optionally substituted with one to three halogen atom(s), and a chlorine atom and a trifluoromethanesulfonyloxy group can be preferably used.

As the reactive derivative of the compound [IV], a boric acid derivative can be preferably used.

Examples of a group shown by L² include a halogen atom, a lower alkylsulfonyloxy group, an arylsulfonyloxy group and the like, and an iodine atom can be preferably used.

The reaction of the compound [III] with the compound [IV] or a reactive derivative thereof can be conducted by carrying out the coupling reaction under basic condition using a catalyst of palladium complex. Examples of palladium of the palladium complex include [1,1'-bis(diphenylphosphino)ferrocene]dichloropalladium(II), tris(dibenzylideneacetone)dipalladium(0), palladium acetate, tris(triethylphosphine)palladium, bis(triphenylphosphine)dichloropalladium, dichloroethylenediaminepalladium, palladium chloride, palladium carbon and the like, and examples of complexes thereof include complexes solvated with a conventional solvent such as dichloromethane, 1,4-dioxane, tetrahydrofuran, benzene, chloroform etc. In the present invention, these catalysts may be used alone or with a combination of two or more catalysts, and a catalyst prepared in the reaction system may be used without purification. Moreover, the complex described above may be mixed with various ligands in a reaction medium and used in situ. Examples of ligands include a phosphine ligand exemplified with triphenylphosphine, diphenylphosphinoferrocene, and 9,9-dimethyl-4,5-bis(diphenylphosphino)xanthene; and a carbene ligand exemplified with 1,3-bis(2,4,6-trimethylphenyl)imidazolium chloride and 1,3-bis(2,6-triisopropylphenyl)imidazoliun chloride.

Any base which is suitable for the reaction may be used, and for example, cesium carbonate, N,N-diisopropylethylamine, tribasic potassium phosphate, sodium carbonate, potassium carbonate, sodium bicarbonate, potassium bicarbonate, sodium hydroxide, potassium hydroxide, triethylamine, 4-dimethylaminopyridine, triethylenediamine, and 4-methylmorpholine can be used.

Any solvent which does not disturb the reaction can be used and for example, dichloromethane, 1,4-dioxane, methanol, ethanol, tetrahydrofuran, chloroform, 1,2-dichloroethane, toluene, 1,2-dimethoxyethane, pyridine, ethyl acetate, water and a mixture thereof can be preferably used. The reaction may be preferably carried out at room temperature to a boiling point of the solvent.

The reaction between the compound [V] and [VI] can be conducted by carrying out the coupling reaction in the presence of a transition metal such as a palladium complex or copper etc. under basic condition. Examples of palladium of the palladium complex include tris(dibenzylideneacetone)dipalladium(0), palladium acetate, [1,1'-bis(diphenylphosphino)ferrocene]dichloropalladium(II), bis(tri-t-butylphosphine)palladium(0) and the like, and the reaction can be preferably carried out by using the complex alone or adding various ligands to the reaction medium. Examples of ligands include a phosphine ligand exemplified with triphenylphosphine, diphenylphosphinoferrocene, and 9,9-dimethyl-4,5-bis(diphenylphosphino)xanthene; and a carbene ligand exemplified with 1,3-bis(2,4,6-trimethylphenyl)imidazolium chloride and 1,3-bis(2,6-triisopropylphenyl)imidazolium chloride. As the copper metal, the reaction can be preferably carried out by using copper iodide etc.

Any base which is suitable for the reaction may be used, and for example, cesium carbonate, tribasic potassium phosphate, sodium carbonate, potassium carbonate, sodium bicarbonate, potassium bicarbonate, sodium hydroxide, potassium hydroxide, triethylamine, triethylenediamine, potassium t-butoxide, sodium t-butoxide, sodium phenoxide, lithium hexamethyldisilazide(LiHMDS) can be used.

The reaction can be preferably carried out at room temperature to heated condition.

Further, the present reaction can be carried out in a suitable solvent under the presence of acid or deacidification agent, or without a solvent. As the acid, a mineral acid such as hydrochloric acid, sulfuric acid, phosphoric acid, hydrobromic acid etc.; an organic acid such as acetic acid, methanesulfonic acid, trifluoromethanesulfonic acid, benzensulfonic acid, tosyl acid etc. and a Lewis acid such as trifluoroborane diehyl ether complex, titanium tetrachloride etc. can be preferably used.

Any solvent which does not disturb the reaction can be used and for example, water, chloroform, dichloromethane, 1,2-dichloroethane, 1,4-dioxane, N-methyl-2-pyrrolidinone, tetrahydrofuran, ethyl acetate, dimethylsulfoxide, methanol, ethanol, butanol, and the like can be preferably used.

The present reaction can be carried out at room temperature to heated condition.

As the deacidification agent, sodium hydride, potassium t-butoxide, lithium diisopropylamide (LDA), sodium hexamethyldisilazide(NaHMDS), potassium hexamethyldisilazide(KHMDS), lithium hexamethyldisilazide(LiMDS) etc. can be preferably used. Any solvent which does not disturb the reaction can be used and for example, N,N-dimethylformamide, tetrahydrofuran, dimethylsulfoxide, chloroform, dichloromethane, 1,2-dichloroethane etc. can be preferably used. The reaction can be carried out at a temperature under ice-cooling to heating.

The reaction between the compound [VII] and [VIII] can be conducted according to the conventional alkylation reaction, and carried out by reacting an alkylating agent(for example, alkyl halide, alkyl sulfonate, alkyl sulfate etc.) with the compound [VII] in a appropriate solvent under the presence of a base.

Example of the base include an inorganic base such as sodium carbonate, potassium carbonate, sodium bicarbonate; an aromatic amine such as pyridine and lutidine; a tertiary amine such as triethylamine, 4-dimethylaminopyridine; an alkali metal hydride such as sodium hydride, potassium hydride; a metal amide such as sodium amide, lithium diiropropylamide, lithium hexamethyldisilazide; and a metal alkoxide such as sodium methoxide, sodium ethoxide, potassium t-butoxide and the like.

Any solvent which does not disturb the reaction can be used and for example, diethyl ether, tetrahydrofuran, 1,4-dioxane, 1,2-dimethoxyethane, toluene, cyclohexane, hexane, N,N-dimethylformamide, N,N-dimethylacetamide, dimethylsulfoxide, 1,3-dimethyl-2-imidazolidinone, N-methyl-2-pyrrolidinone, dichloromethane, chloroform, carbontetrachloride, 1,2-dichloroethane, acetonitrile, propionitrile and a mixture thereof are preferable. The reaction can be carried out at a temperature under ice-cooling to heating.

### [Reaction of Method B]

As a protecting group shown by P², a benzyl group, an ethyl group and a methyl group can be preferably used for example.

Examples of X³ include a halogen atom, an alkyl sulfonate group, a lower alkylthio group, a lower alkyl sulfinyl group, and a lower alkyl sulfonyl group optionally substituted with one to three halogen atom(s), and a chlorine atom can be preferably used.

The reaction between the compound [III] and [IX] can be carried out by the reaction in an appropriate solvent under the presence of a base. Examples of the base include potassium carbonate, sodium carbonate, sodium hydride and potassium hydride etc., and preferably sodium hydride. Examples of the solvent include any solvent which does not disturb the reaction, such as diethyl ether, tetrahydrofuran, 1,4-dioxane, 1,2-dimethoxyethane, toluene, cyclohexane, hexane, N,N-dimethylformamide, N,N-dimethylacetamide, dimethylsulfoxide, 1,3-dimethyl-2-imidazolidinone, N-methyl-2-pyrrolidinone, dichloromethane, chloroform, carbontetrachloride, 1,2-dichloroethane, acetonitrile, propionitrile etc. and a mixture thereof. The present reaction can be conducted preferably at a temperature under cooling to heating.

The reaction between the compound [X] and [VI] can be carried out in the same manner as the reaction between the compound [V] and [VI].

The reaction between the compound [XI] and [VIII] can be carried out in the same manner as the reaction between the compound [VII] and [VIII].

The deprotection of P² group in the compound [XII] can be conducted in the same manner as that of the protecting group P¹ of the compound [II]

As to conversion to a leaving group conducted in the next step, halogenation is preferable and it can be carried out by reacting a halogenating agent in an inert solvent or without a solvent. Examples of the halogenating agent include phosphorus oxychloride, phosphorus oxybromide, thionyl bromide, thionyl chloride and the like, and phosphorus oxychloride is preferably used.

Examples of the solvent include any solvent which does not disturb the reaction, such as toluene, methylenechloride, chloroform, carbontetrachloride, dichloroethane, N,N-dimethylformamide, and the like. The reaction can be conducted preferably at -20°C to 100°C.

The reaction between the compound [XIII] and the compound [IV] or reactive intermediate thereof can be conducted in the same manner as the reaction between the compound [III] and the compound [IV] or reactive intermediate thereof.

The resulting compound [I] can be mutually converted if necessary and the mutual conversion can be conducted by a conventional method such as halogenation, alkylation, nitration, amination, reduction, oxidation, de-alkylation and the like.

### Best Mode for Carrying Out the Invention

Examples of the objective compounds of the present invention prepared by using each method illustrated above are shown below, but the present invention should not be construed to be limited thereto.

### Example 1

### N-[2-Chloro-4-(trifluoromethyl)phenyl]-N-ethyl-7-(2-fluoro-6-methoxyphenyl)-1H-pyrazolo[4,3-d]pyrimidine-5-amine

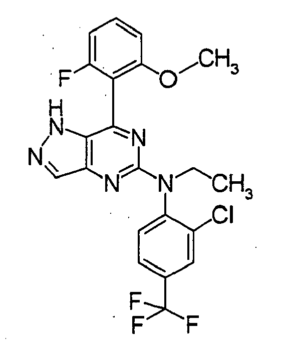

N-[2-Chloro-4-(trifluoromethyl)phenyl]-N-ethyl-7-(2-fluoro-6-methoxyphenyl)-2-(4-methoxybenzyl)-2H-pyrazolo[4,3-d]pyrimidine-5-amine(7.73 g) (a compound obtained in Reference Example 11 or 17) was dissolved in trifluoroacetic acid(40 mL) and heated to reflux under argon atmosphere overnight. After being cooled to room temperature, the reaction mixture was concentrated under reduced pressure and a saturated aqueous solution of sodium bicarbonate and ethyl acetate were added. The organic layer was separated and washed with a saturated brine, dried over sodium sulfate, and the solvent was evaporated under reduced pressure. The resulted residue was purified by using a silica gel column chromatography (hexane:ethyl acetate=3:1 to 3:2) to give a crude crystalline, which was recrystallized from a mixture of hexane and ethyl acetate to give the titled compound(6.08 g, 81% yield) as a pale yellow crystalline.
mp.184-185°C, MS(APCI)m/z:466/468[M+H]⁺.

### Example 2

### N-[2-Chloro-4-(trifluoromethyl)phenyl]-7-[2-(cyclopropylmethoxy)phenyl]-N-ethyl-1H-pyrazolo[4,3-d]pyrimidine-5-amine

To a solution of N-[2-chloro-4-(trifluoromethyl)phenyl]-7-[2-(cyclopropylmethoxy)phenyl]-N-ethyl-2-(4-methoxybenzyl)-2H-pyrazolo[4,3-d]pyrimidine-5-amine(51 mg) in N,N-dimethylformamide(2 mL), was added sodium hydride(20 mg) at room temperature and the mixture was stirred overnight under argon atmosphere. Water was poured into the reaction mixture and it was extracted with ethyl acetate. The organic extraction layer was washed with water and a saturated brine, dried over sodium sulfate and the solvent was evaporated under reduced pressure. The resulted residue was purified by using a silica gel column chromatography (hexane:ethyl acetate=9:1 1 to 3:2) to give the titled compound(22.8 mg, 58% yield) as an amorphous powder. MS(APCI)m/z:488/490[M+H]⁺.

### Example 3

### (1)Ethyl [5-[N-[2-chloro-4-(trifluoromethyl)phenyl]-ethylamino]-2-(4-methoxybenzyl)-1H-pyrazolo[4,3-d]pyrimidine-7-yl](pyridine-3-yl)acetate

To a solution of ethyl 3-pyridyl acetate(203.8 mg) in N,N-dimethylformamide(1.5 mL), was added sodium hydride(60% in oil; 48.4 mg) and the mixture was stirred at room temperature for 15 minutes. To the reaction mixture was added 7-chloro-N-[2-chloro-4-(trifluoromethyl)phenyl]-N-ethyl-2-(4-methoxybenzyl)-2H-pyrazolo[4,3-d]pyrimidine-5-amine(300 mg) (a compound obtained in reference example 16) dissolved in N,N-dimethylformamide(1.5 mL) and the mixture was stirred at 80°C for 50 minutes. A saturated aqueous solution of ammonium chloride was added under ice cooling and the product was extracted with ethyl acetate. The organic extraction layer was dried over sodium sulfate and the solvent was evaporated under reduced pressure. The residue was purified by using NH silica gel column chromatography (hexane:ethyl acetate=1:0 to 1:1) to give a titled compound(353.6 mg, 93.6% yield) as a brown viscous oil. MS(APCI)m/z:625/627[M+H]⁺.

### (2)Ethyl [5-[N-[2-chloro-4-(trifluoromethyl)phenyl]-ethylamino]-1H-pyrazolo[4,3-d]pyrimidine-7-yl](pyridine-3-yl)acetate

Ethyl [5-[N-[2-chloro-4-(trifluoromethyl)phenyl]-ethylamino]-2-(4-methoxybenzyl)-1H-pyrazolo[4,3-d]pyrimidine-7-yl](pyridine-3-yl)acetate (353.6 mg)(a compound obtained in (1) above) was treated in a similar manner to the method of Example 1 to give the titled compound(197.8 mg, 69.3% yield) as a yellow amorphous powder. MS(APCI)m/z:505/507[M+H]⁺.

### (3)N-[2-Chloro-4-(trifluoromethyl)phenyl]-N-ethyl-7-(pyridine-3-ylmethyl)-1H-pyrazolo[4,3-d]pyrimidine-5-amine

To a solution of ethyl [5-[N-[2-chloro-4-(trifluoromethyl)phenyl]-ethylamino]-1H-pyrazolo[4,3-d]pyrimidine-7-yl](pyridine-3-yl)acetate(a compound obtained in (2) above) (100 mg) in ethanol (2 mL), was added an aqueous 2N NaOH solution(0.3 mL) and the mixture was stirred at 80°C for 40 minutes. After being cooled to room temperature, the solution was neutralized by adding an aqueous 2N HCl solution (0.3 mL) and concentrated under reduced pressure. The resulted residue was purified by using a silica gel column chromatography (chloroform:methanol=10:1) to give the titled compound(11.0 mg, 12.8% yield) as a brown viscous oil.
MS(APCI)m/z:433/435[M+H]⁺.

### Example 4

### (1)N-[2-Chloro-4-(trifluoromethyl)phenyl]-N-ethyl-7-{2-fluoro-6-[2-(tetrahydro-2H-pyrane-2-yloxy)ethoxy]phenyl}-2-(4-methoxybenzyl)-2H-pyrazolo[4,3-d]pyrimidine-5-amine

Cyanomethylene-N-butylphosphorane(176 mg) was added to a mixture of N-[2-chloro-4-(trifluoromethyl)phenyl]-N-ethyl-7-(2-fluoro-6-hydroxyphenyl)-2-(4-methoxybenzyl)-1H-pyrazolo[4,3-d]pyrimidine-5-amine(a compound obtained in reference example 11 or 17) (250 mg) and 2- (2-hydroxyethoxy)tetrahydrofuran(102.3 mg) dissolved in toluene(2.0 mL) at room temperature under argon atmosphere and the mixture was heated to reflux overnight. After being cooled to room temperature, the reaction mixture was concentrated under reduced pressure and the resulted residue was purified by using a silica gel column chromatography (hexane:ethyl acetate=3:1 to 1:1) to give the titled compound(293 mg, 95% yield)as a light brown oil. MS(APCI) m/z:700/702[M+NH₄]⁺.

### (2)N-[2-Chloro-4-(trifluoromethyl)phenyl]-N-ethyl-7-{2-fluoro-6-[2-(tetrahydro-2H-pyrane-2-yloxy)ethoxy]phenyl}-1H-pyrazolo[4,3-d]pyrimidine-5-amine

Sodium hydride(33 mg) was added to N-[2-Chloro-4-(trifluoromethyl)phenyl]-N-ethyl-7-{2-fluoro-6-[2-(tetrahydro-2H-pyran-2-yloxy)ethoxy]phenyl}-2-(4-methoxybenzyl)-2H-pyrazolo[4,3-d]pyrimidine-5-amine(a compound obtained in (1) above) (114 mg) dissolved in N,N-dimethylformamide(3.0 mL) under argon atmosphere and the mixture was stirred at room temperature overnight. Water was poured into the mixture and the product was extracted with ethyl acetate. The organic extraction layer was washed with water and a saturated brine, dried over sodium sulfate and the solvent was evaporated under reduced pressure. The resulted residue was purified by using NH silica gel column chromatography (hexane:ethyl acetate=3:1 to 5:3) to give the titled compound(328 mg) as a colorless oil quantitatively. (APCI)m/z:580/582[M+H]⁺. (3)2-(2-{5-[[2-Chloro-4-(trifluoromethyl)phenyl (ethyl)amino]-1H-pyrazolo[4,3-d]pyrimidine-7-yl}-3-fluorophenoxy)ethanol

5% aqueous HCl(2.0 mL) was added to N-[2-Chloro-4-(trifluoromethyl)phenyl]-N-ethyl-7-{2-fluoro-6-[2-(tetrahydro-2H-pyrane-2-yloxy)ethoxy]phenyl}-1H-pyrazolo[4,3-d]pyrimidine-5-amine(a compound obtained in (2) above) (320 mg) dissolved in tetrahydrofuran(2.0 mL) and the mixture was stirred for 10 minutes. A saturated aqueous solution of sodium bicarbonate was poured into the reaction mixture and the product was extracted with ethyl acetate. The organic extraction layer was washed with water and a saturated brine, dried over sodium sulfate and the solvent was evaporated under reduced pressure. The resulted residue was purified by using a silica gel column chromatography (hexane:ethyl acetate=1:1 to 1:2) to give the titled compound(13.6 mg, 17% yield) as a colorless oil.
(APCI)m/z:496/498[M+H]⁺.

### Example 5-178

Compounds in the table 1-23 below were obtained by treating the corresponding compound in the similar manner to any of examples.

**Table 1**

| | | | |
|---|---|---|---|
| 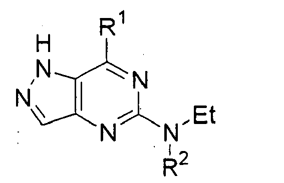 | | | |

| Examples | R¹ | R² | Physical Constants |
|---|---|---|---|
| 5 | 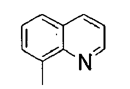 | 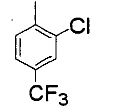 | Crystalline MS(APCI) m/z:469/471[M+H]⁺ |
| 6 | 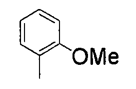 | 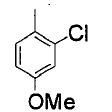 | Amorphous MS(APCI) m/z:410/412[M+H]⁺ |
| 7 | 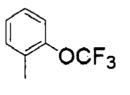 | 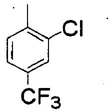 | Crystalline MS(APCI) m/z:502/504[M+H]⁺ |
| 8 | 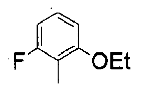 | 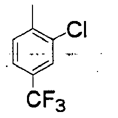 | Crystalline MS(APCI) m/z:480/482[M+H]⁺ |
| 9 | 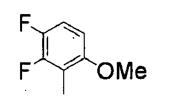 | 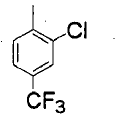 | Amorphous MS(APCI) m/z:484/486[M+H]⁺ |
| 10 | 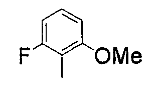 | 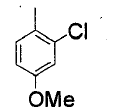 | Amorphous MS (APCI) m/z:428[M+H]⁺ |
| 11 | 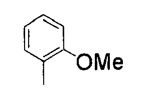 | 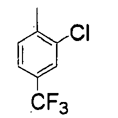 | Amorphous MS(APCI) m/z:448/450[M+H]⁺ |
| 12 | 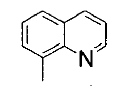 | 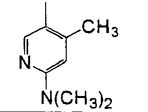 | Amorphous MS(APCI) m/z:425[M+H]⁺ |

**Table 2**

| | | | |
|---|---|---|---|
| 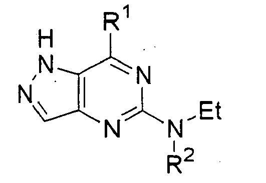 | | | |

| Examples | R¹ | R² | Physical Constants |
|---|---|---|---|
| 13 | 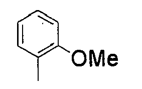 | 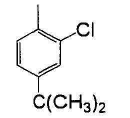 | Amorphous MS(APCI) m/z:422/424[M+H]⁺ |
| 14 | 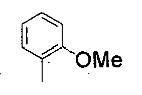 | 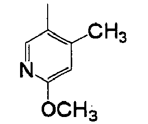 | Amorphous MS(APCI) m/z:391[M+H]⁺ |
| 15 | 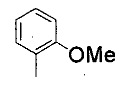 | 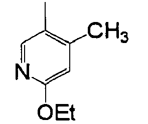 | Amorphous MS(APCI) m/z:405[M+H]⁺ |
| 16 | 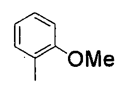 | 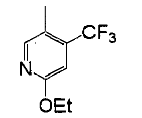 | Amorphous MS(APCI) m/z:459[M+H]⁺ |
| 17 | 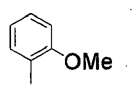 | 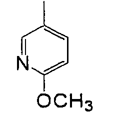 | Amorphous MS(APCI) m/z:377[M+H]⁺ |
| 18 | 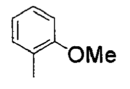 | 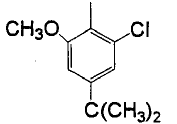 | Amorphous MS(APCI) m/z:452/454[M+H]⁺ |
| 19 | 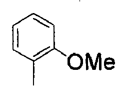 | 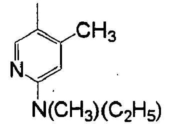 | Amorphous MS(APCI) m/z:418[M+H]⁺ |
| 20 | 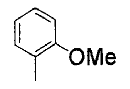 | 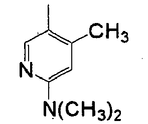 | Amorphous MS (APCI) m/z:404[M+H]⁺ |

**Table 3**

| | | | |
|---|---|---|---|
| 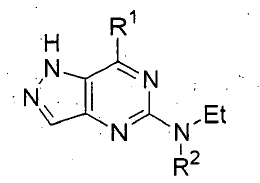 | | | |

| Examples | R¹ | R² | Physical Constants |
|---|---|---|---|
| 21 | 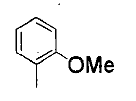 | 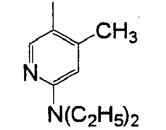 | Amorphous MS(APCI) m/z:432[M+H]⁺ |
| 22 | 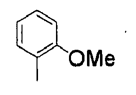 | 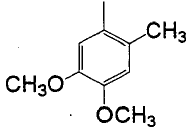 | Amorphous MS (APCI) m/z:420[M+H]⁺ |
| 23 | 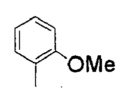 | 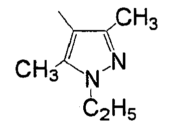 | Amorphous MS(APCI) m/z:392[M+H]⁺ |
| 24 | 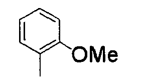 | 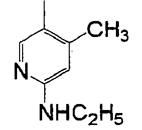 | Amorphous MS(APCI) m/z:404[M+H]⁺ |
| 25 | 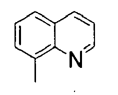 | 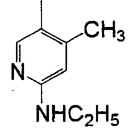 | Amorphous MS(APCI) m/z:425[M+H]⁺ |
| 26 | 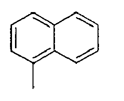 | 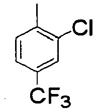 | Amorphous MS(APCI) m/z:468/470[M+H]⁺ |
| 27 | 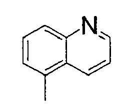 | 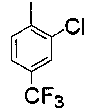 | Amorphous MS(APCI) m/z:469/471[M+H]⁺ |
| 28 | 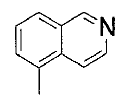 | 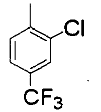 | Amorphous MS(APCI) m/z:469/471[M+H]⁺ |

**Table 4**

| | | | |
|---|---|---|---|
| 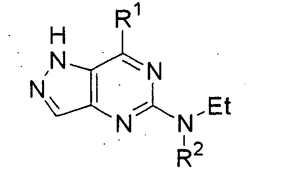 | | | |

| Examples | R¹ | R² | Physical Constants |
|---|---|---|---|
| 29 | 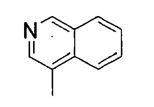 | 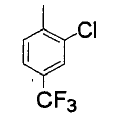 | Amorphous MS(APCI) m/z:469/471[M+H]⁺ |
| 30 | 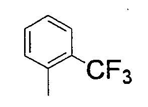 | 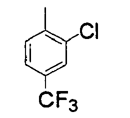 | Amorphous MS(APCI) m/z:486/488[M+H]⁺ |
| 31 | 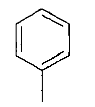 | 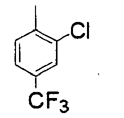 | Amorphous MS(APCI) m/z:418/420[M+H]⁺ |
| 32 | 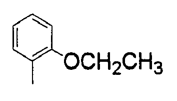 | 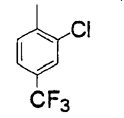 | Amorphous MS(APCI) m/z:462/464[M+H]⁺ |
| 33 | 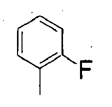 | 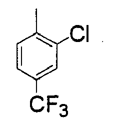 | Amorphous MS(APCI) m/z:436/438[M+H]⁺ |
| 34 | 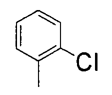 | 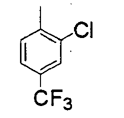 | Amorphous MS(APCI) m/z:452/454[M+H]⁺ |
| 35 | 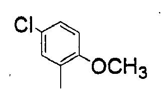 | 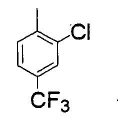 | Amorphous MS(APCI) m/z:482/484[M+H]⁺ |
| 36 | 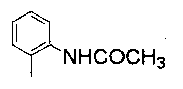 | 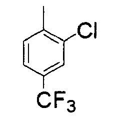 | Amorphous MS(APCI) m/z:475/477[M+H]⁺ |

**Table 5**

| | | | |
|---|---|---|---|
| 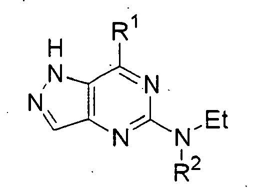 | | | |

| Examples | R¹ | R ² | Physical Constants |
|---|---|---|---|
| 37 | 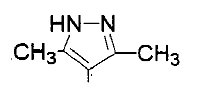 | 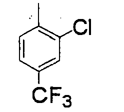 | Amorphous MS(APCI) m/z:436/438[M+H]⁺ |
| 38 | 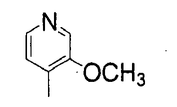 | 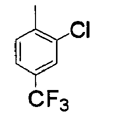 | Amorphous MS(APCI) m/z:449/451[M+H]⁺ |
| 39 | 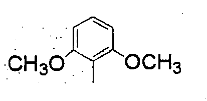 | 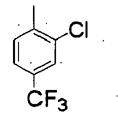 | Amorphous MS(APCI) m/z:478/480[M+H]⁺ |
| 40 | 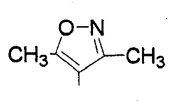 | 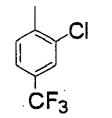 | Amorphous MS(APCI) m/z:437/439[M+H]⁺ |
| 41 | 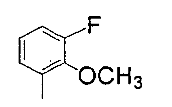 | 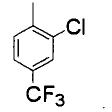 | Amorphous MS(APCI) m/z:466/468[M+H]⁺ |
| 42 | 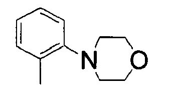 | 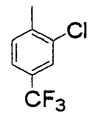 | Amorphous MS(APCI) m/z:503/505[M+H]⁺ |
| 43 | 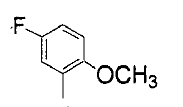 | 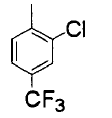 | Amorphous MS(APCI) m/z:466/468[M+H]⁺ |
| 44 | 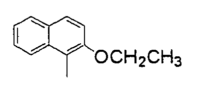 | 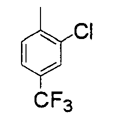 | Amorphous MS(APCI) m/z:512/514[M+H]⁺ |

**Table 6**

| | | | |
|---|---|---|---|
| 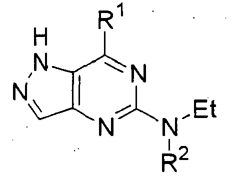 | | | |

| Examples | R¹ | R² | Physical Constants |
|---|---|---|---|
| 45 | 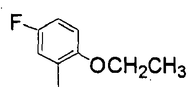 | 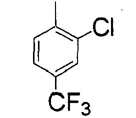 | Amorphous MS(APCI) m/z:480/482[M+H]⁺ |
| 46 | 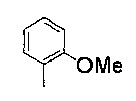 | 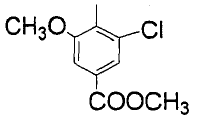 | Amorphous MS(APCI) m/z:468/470[M+H]⁺ |
| 47 | 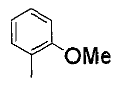 | 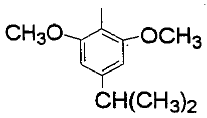 | Amorphous MS(APCI) m/z:448[M+H]⁺ |
| 48 | 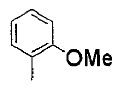 | 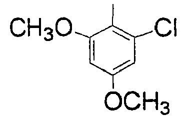 | Amorphous MS(APCI) m/z:440/442[M+H]⁺ |
| 49 | 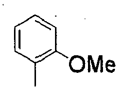 | 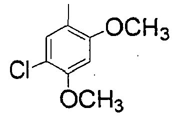 | Amorphous MS (APCI) m/z:440/442[M+H]⁺ |
| 50 | 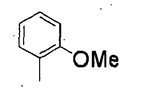 | 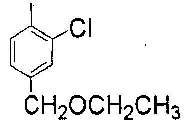 | Amorphous MS(APCI) m/z:438/440[M+H]⁺ |
| 51 | 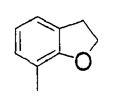 | 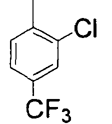 | Amorphous MS(APCI) m/z:460/462[M+H]⁺ |
| 52 | 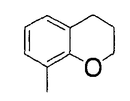 | 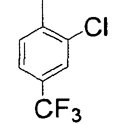 | Amorphous MS(APCI) m/z:474/476[M+H]⁺ |

**Table 7**

| | | | |
|---|---|---|---|
| 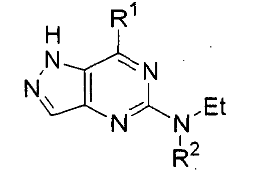 | | | |

| Examples | R¹ | R² | Physical Constants |
|---|---|---|---|
| 53 | 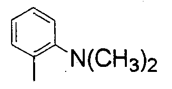 | 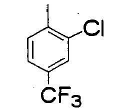 | Amorphous MS(APCI) m/z:461/463[M+H]⁺ |
| 54 | 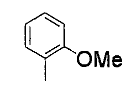 | 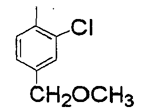 | Amorphous MS(APCI) m/z:424/426[M+H]⁺ |
| 55 | 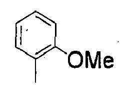 | 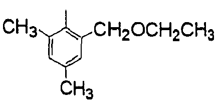 | Amorphous MS(APCI) m/z:432[M+H]⁺ |
| 56 | 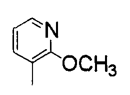 | 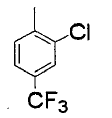 | Amorphous MS(APCI) m/z:449/451[M+H]⁺ |
| 57 | 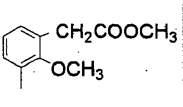 | 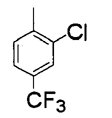 | Amorphous MS(APCI) m/z:520/522[M+H]⁺ |
| 58 | 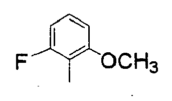 | 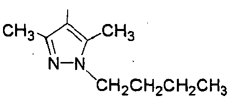 | Amorphous MS(APCI) m/z:438[M+H]⁺ |
| 59 | 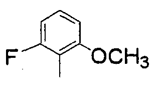 | 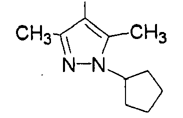 | Amorphous MS(APCI) m/z:450[M+H]⁺ |
| 60 | 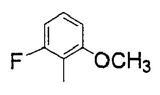 | 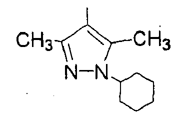 | Amorphous MS(APCI) m/z:464[M+H]⁺ |

**Table 8**

| | | | |
|---|---|---|---|
| 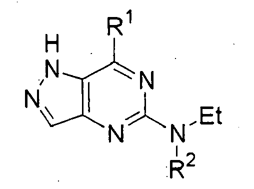 | | | |

| Examples | R¹ | R² | Physical Constants |
|---|---|---|---|
| 61 | 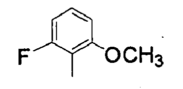 | 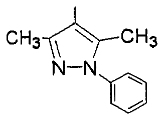 | Amorphous MS(APCI) m/z:458[M+H]⁺ |
| 62 | 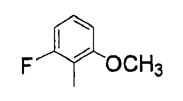 | 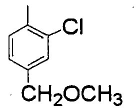 | Amorphous MS(APCI) m/z:442/444[M+H]⁺ |
| 63 | 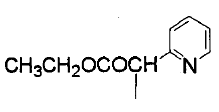 | 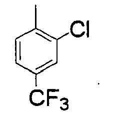 | Amorphous MS(APCI) m/z:505/507[M+H]⁺ |
| 64 | 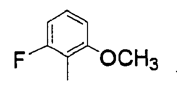 | 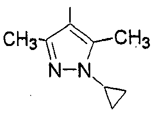 | Amorphous MS(APCI) m/z:422[M+H]⁺ |
| 65 | 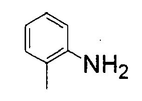 | 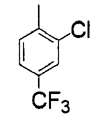 | Amorphous MS(APCI) m/z:433/435[M+H]⁺ |
| 66 | 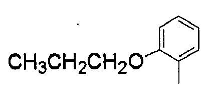 | 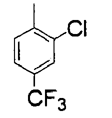 | Amorphous MS(ESI+) m/z:476[M+H]⁺ |
| 67 | 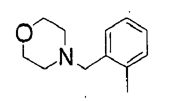 | 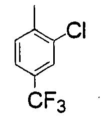 | Amorphous MS(ESI+) m/z:517[M+H]⁺ |
| 68 | 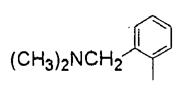 | 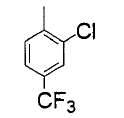 | Amorphous MS (ESI+) m/z:475[M+H]⁺ |

**Table 9**

| | | | |
|---|---|---|---|
| 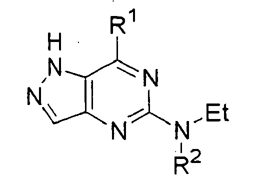 | | | |

| Examples | R¹ | R² | Physical Constants |
|---|---|---|---|
| 69 | 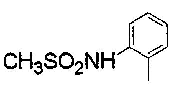 | 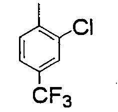 | Amorphous MS(APCI) m/z:511/513[M+H]⁺ |
| 70 | 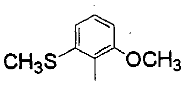 | 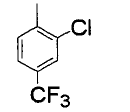 | Amorphous MS(APCI) m/z:494/496[M+H]⁺ |
| 71 | 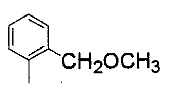 | 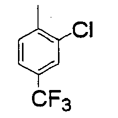 | Amorphous MS(APCI) m/z:462/464[M+H]⁺ |
| 72 | 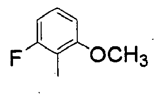 | 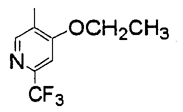 | Amorphous MS(APCI) m/z:477[M+H]⁺ |
| 73 | 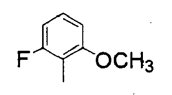 | 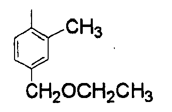 | Amorphous MS(APCI) m/z:436[M+H]⁺ |
| 74 | 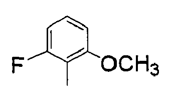 | 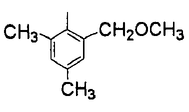 | Amorphous MS(APCI) m/z:436[M+H]⁺ |
| 75 | 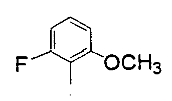 | 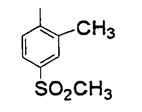 | Amorphous MS (APCI) m/z:456[M+H]⁺ |
| 76 | 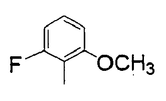 | 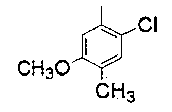 | Amorphous MS(APCI) m/z:442/444[M+H]⁺ |

**Table 10**

| | | | |
|---|---|---|---|
| 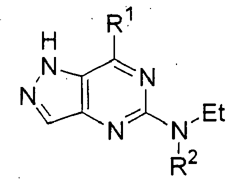 | | | |

| Examples | R¹ | R² | Physical Constants |
|---|---|---|---|
| 77 | 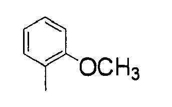 | 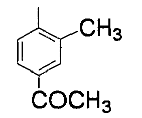 | Amorphous MS (APCI) m/z:402[M+H]⁺ |
| 78 | 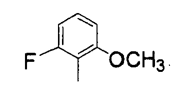 | 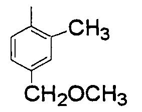 | Amorphous MS(APCI) m/z:422[M+H]⁺ |
| 79 | 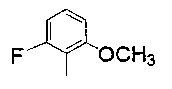 | 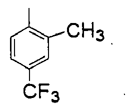 | Amorphous MS(APCI) m/z:446[M+H]⁺ |
| 80 | 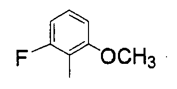 | 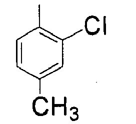 | Amorphous MS(APCI) m/z:412/414[M+H]⁺ |
| 81 | 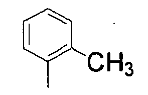 | 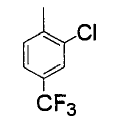 | Amorphous MS(APCI) m/z:432/434[M+H]⁺ |
| 82 | 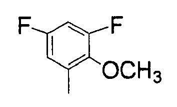 | 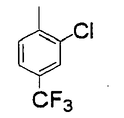 | Amorphous MS(APCI) m/z:484/486[M+H]⁺ |
| 83 | 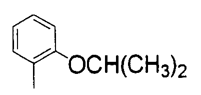 | 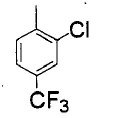 | Amorphous MS(APCI) m/z:476/478[M+H]⁺ |
| 84 | 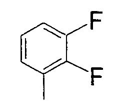 | 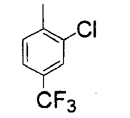 | Amorphous MS(APCI) m/z:454/456[M+H]⁺ |

**Table 11**

| | | | |
|---|---|---|---|
| 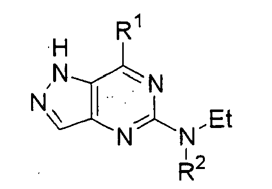 | | | |

| Examples | R¹ | R² | Physical Constants |
|---|---|---|---|
| 85 | 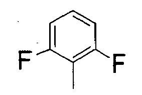 | 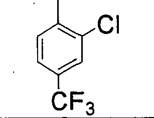 | Amorphous MS(APCI) m/z:454/456[M+H]⁺ |
| 86 | 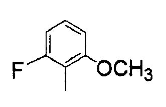 | 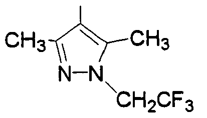 | Amorphous MS(APCI) m/z:464[M+H]⁺ |
| 87 | 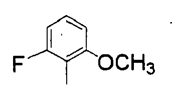 | 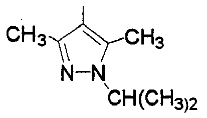 | Amorphous MS(APCI) m/z:424[M+H]⁺ |
| 88 | 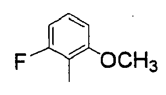 | 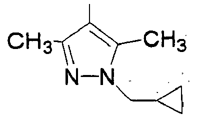 | Amorphous MS(APCI) m/z:436[M+H]⁺ |
| 89 | 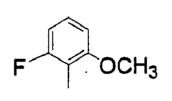 | 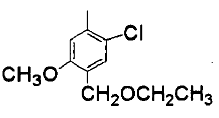 | Amorphous MS(APCI) m/z:486/488[M+H]⁺ |
| 90 | 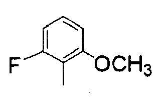 | 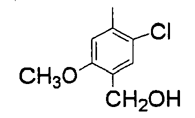 | Amorphous MS(APCI) m/z:458/460[M+H]⁺ |
| 91 | 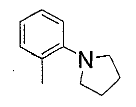 | 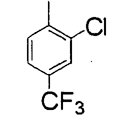 | Amorphous MS(APCI) m/z:487/489[M+H]⁺ |
| 92 | 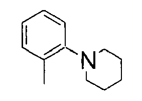 | 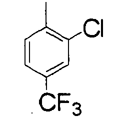 | Amorphous MS(APCI) m/z:501/503[M+H]⁺ |

**Table 12**

| | | | |
|---|---|---|---|
| 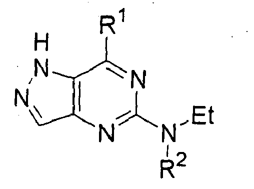 | | | |

| Examples | R¹ | R² | Physical Constants |
|---|---|---|---|
| 93 | 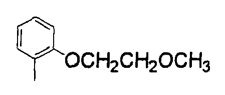 | 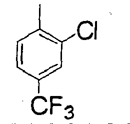 | Amorphous MS (APCI) m/z:492/494[M+H]⁺ |
| 94 | 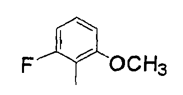 | 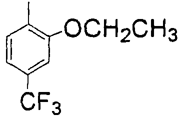 | Amorphous MS(APCI) m/z:476[M+H]⁺ |
| 95 | 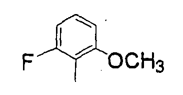 | 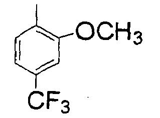 | Amorphous MS(APCI) m/z:462[M+H]⁺ |
| 96 | 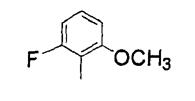 | 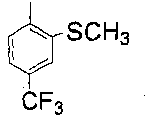 | Amorphous MS(APCI) m/z:478[M+H]⁺ |
| 97 | 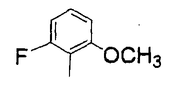 | 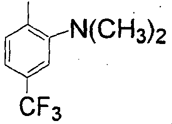 | Amorphous MS (APCI) m/z:475[M+H]⁺ |
| 98 | 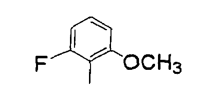 | 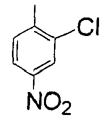 | Amorphous MS(APCI) m/z:443/445[M+H]⁺ |
| 99 | 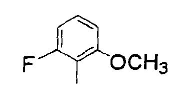 | 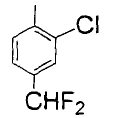 | Amorphous MS(APCI) m/z:448/450[M+H]⁺ |
| 100 | 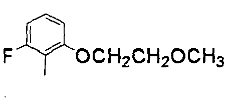 | 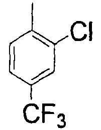 | Amorphous MS(APCI) m/z:510/512[M+H]⁺ |

**Table 13**

| | | | |
|---|---|---|---|
| 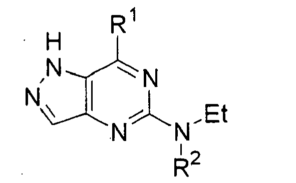 | | | |

| Examples | R¹ | R² | Physical Constants |
|---|---|---|---|
| 101 | 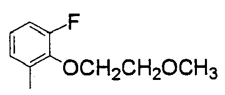 | 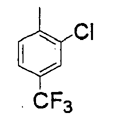 | Amorphous MS(APCI) m/z:510/512[M+H]⁺ |
| 102 | 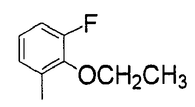 | 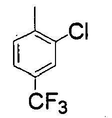 | Amorphous MS(APCI) m/z:480/482[M+H]⁺ |
| 103 | 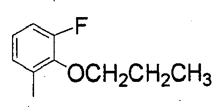 | 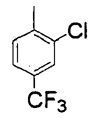 | Amorphous MS(APCI) m/z:494/496[M+H]⁺ |
| 104 | 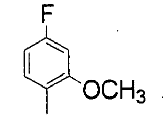 | 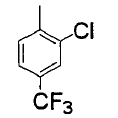 | Amorphous MS(APCI) m/z:466/468[M+H]⁺ |
| 105 | 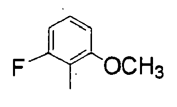 | 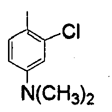 | Amorphous MS(APCI) m/z:441/443[M+H]⁺ |
| 106 | 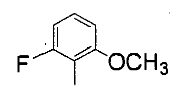 | 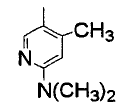 | Amorphous MS (APCI) m/z:422[M+H]⁺ |
| 107 | 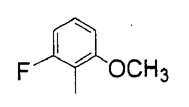 | 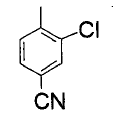 | Amorphous MS(APCI) m/z:423/425[M+H]⁺ |
| 108 | 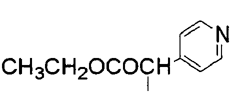 | 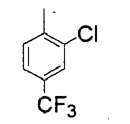 | Amorphous MS(APCI) m/z:505/507[M+H]⁺ |

**Table 14**

| | | | |
|---|---|---|---|
| 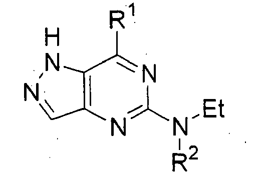 | | | |

| Examples | R¹ | R² | Physical Constants |
|---|---|---|---|
| 109 | 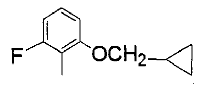 | 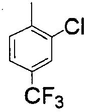 | Amorphous MS(APCI) m/z:506/508[M+H]⁺ |
| 110 | 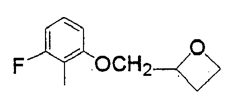 | 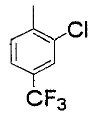 | Amorphous MS(APCI) m/z:522/524[M+H]⁺ |
| 111 | 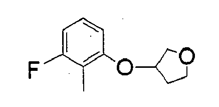 | 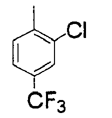 | Amorphous MS(APCI) m/z:522/524[M+H]⁺ |
| 112 | 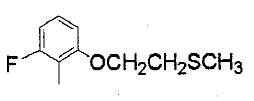 | 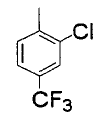 | Amorphous MS(APCI) m/z:526/528[M+H]⁺ |
| 113 | 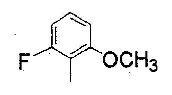 | 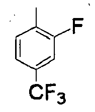 | Amorphous MS(APCI) m/z:450[M+H]⁺ |
| 114 | 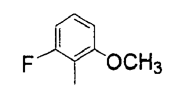 | 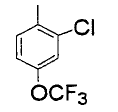 | Amorphous MS(APCI) m/z:482/484[M+H]⁺ |
| 115 | 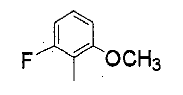 | 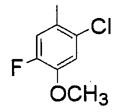 | Amorphous MS(APCI) m/z:446/448[M+H]⁺ |
| 116 | 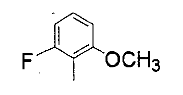 | 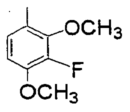 | Amorphous MS(APCI) m/z:442[M+H]⁺ |

**Table 15**

| | | | |
|---|---|---|---|
| 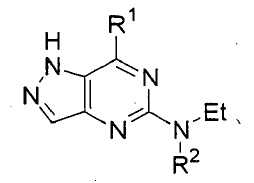 | | | |

| Examples | R¹ | R ² | Physical Constants |
|---|---|---|---|
| 117 | 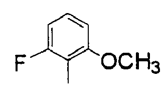 | 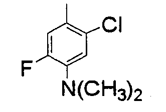 | Amorphous MS(APCI) m/z:459/461[M+H]⁺ |
| 118 | 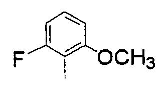 | 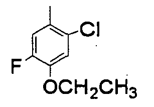 | Amorphous MS(APCI) m/z:460/462[M+H]⁺ |
| 119 | 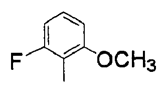 | 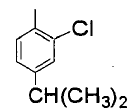 | Amorphous MS(APCI) m/z:440/442[M+H]⁺ |
| 120 | 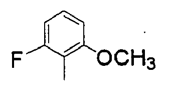 | 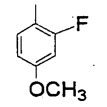 | Amorphous MS(APCI) m/z:412[M+H]⁺ |
| 121 | 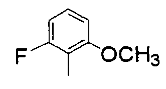 | 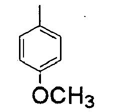 | Amorphous MS(APCI) m/z:394[M+H]⁺ |
| 122 | 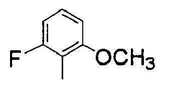 | 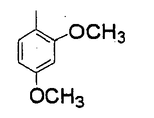 | Amorphous MS(APCI) m/z:424[M+H]⁺ |
| 123 | 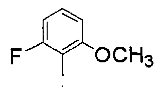 | 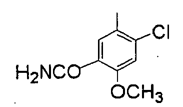 | Amorphous MS(APCI) m/z:471/473[M+H]⁺ |
| 124 | 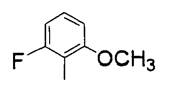 | 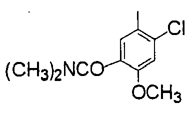 | Amorphous MS(APCI) m/z:499/5017[M+H]⁺ |

**Table 16**

| | | | |
|---|---|---|---|
| 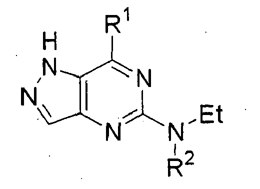 | | | |

| Examples | R¹ | R² | Physical Constants |
|---|---|---|---|
| 125 | 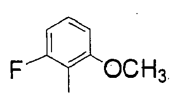 | 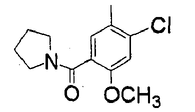 | Amorphous MS (APCI) m/z:525/527[M+H]⁺ |
| 126 | 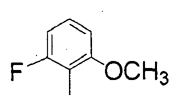 | 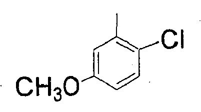 | Amorphous MS(APCI) m/z:428/430[M+H]⁺ |
| 127 | 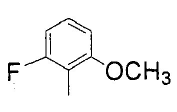 | 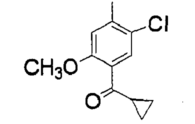 | Amorphous MS(APCI) m/z:496/498[M+H]⁺ |
| 128 | 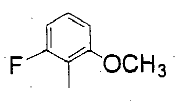 | 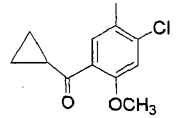 | Amorphous MS(APCI) m/z:496,/498[M+H]⁺ |
| 129 | 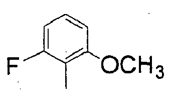 | 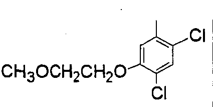 | Amorphous MS(APCI) m/z:506/508[M+H]⁺ |
| 130 | 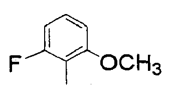 | 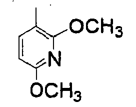 | Amorphous MS(APCI) m/z:425[M+H]⁺ |
| 131 | 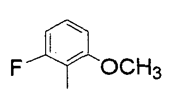 | 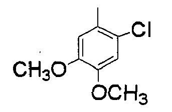 | Amorphous MS(APCI) m/z :458/460[M+H]⁺ |
| 132 | 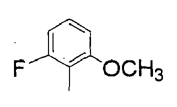 | 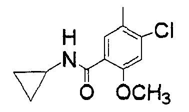 | Amorphous MS(APCI) m/z:511/513[M+H]⁺ |

**Table 17**

| | | | |
|---|---|---|---|
| 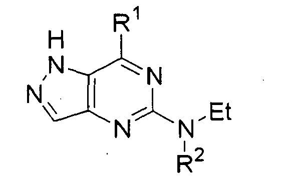 | | | |

| Examples | R¹ | R² | Physical Constants |
|---|---|---|---|
| 133 | 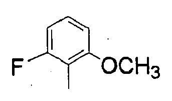 | 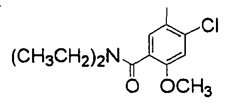 | Amorphous MS(APCI) m/z:527/529[M+H]⁺ |
| 134 | 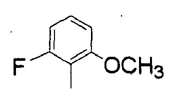 | 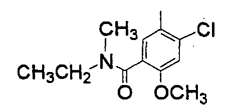 | Amorphous MS(APCI) m/z:513/515[M+H]⁺ |
| 135 | 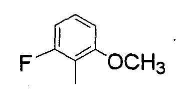 | 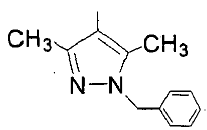 | Amorphous MS(APCI) m/z:472[M+H]⁺ |
| 136 | 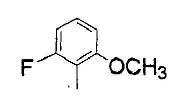 | 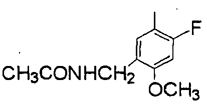 | Amorphous MS(APCI) m/z:483[M+H]⁺ |
| 137 | 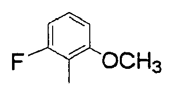 | 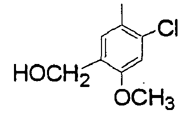 | Amorphous MS(APCI) m/z:458/460[M+H]⁺ |
| 138 | 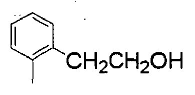 | 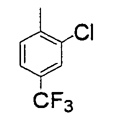 | Amorphous MS(APCI) m/z:462/464[M+H]⁺ |
| 139 | 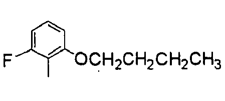 | 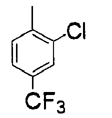 | Amorphous MS(APCI) m/z:508/510[M+H]⁺ |
| 140 | 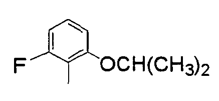 | 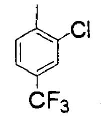 | Amorphous MS(APCI) m/z:494/496[M+H]⁺ |

**Table 18**

| | | | |
|---|---|---|---|
| 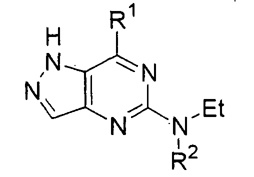 | | | |

| Examples | R¹ | R² | Physical Constants |
|---|---|---|---|
| 141 | 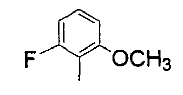 | 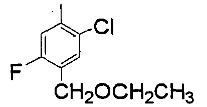 | Amorphous MS(APCI) m/z:474/476[M+H]⁺ |
| 142 | 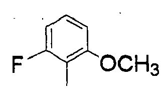 | 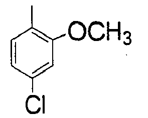 | Amorphous MS(APCI) m/z:428/430[M+H]⁺ |
| 143 | 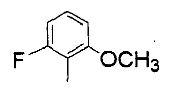 | 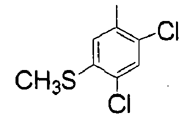 | Amorphous MS(APCI) m/z:478/480[M+H]⁺ |
| 144 | 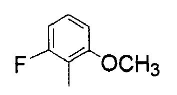 | 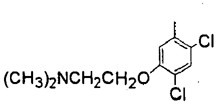 | Amorphous MS (APCI) m/z:519/521[M+H]⁺ |
| 145 | 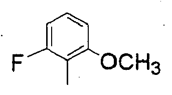 | 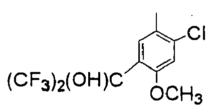 | Amorphous MS (APCI) m/z:594/596[M+H]⁺ |
| 146 | 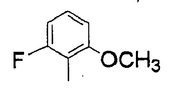 | 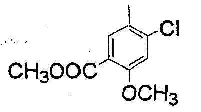 | Amorphous MS(APCI) m/z:486/488[M+H]⁺ |
| 147 | 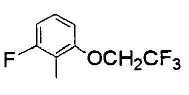 | 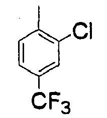 | Amorphous MS(APCI) m/z:534/536[M+H]⁺ |
| 148 | 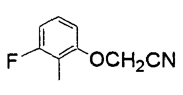 | 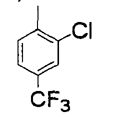 | Amorphous MS(APCI) m/z:491/493[M+H]⁺ |

**Table 19**

| | | | |
|---|---|---|---|
| 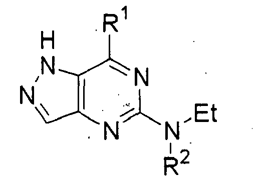 | | | |

| Examples | R¹ | R² | Physical Constants |
|---|---|---|---|
| 149 | 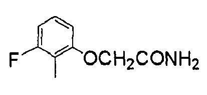 | 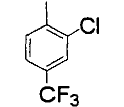 | Amorphous MS(APCI) m/z:509/511[M+H]⁺ |
| 150 | 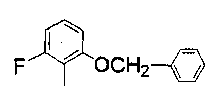 | 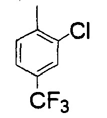 | Amorphous MS(APCI) m/z:/542/544[M+H]⁺ |
| 151 | 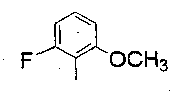 | 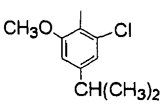 | Amorphous MS(APCI) m/z:470/472[M+H]⁺ |
| 152 | 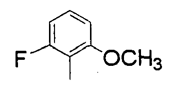 | 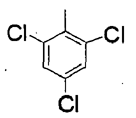 | Amorphous MS(APCI) m%z:466/468[M+H]⁺ |
| 153 | 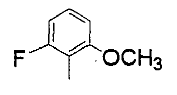 | 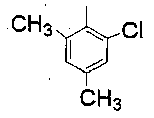 | Amorphous MS(APCI) m/z:426/428[M+H]⁺ |
| 154 | 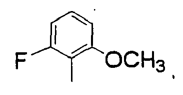 | 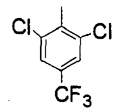 | Amorphous MS(APCI) m/z:500/502[M+H]⁺ |
| 155 | 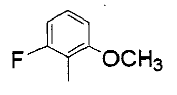 | 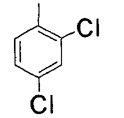 | Amorphous MS(APCI) m/z:432/434[M+H]⁺ |
| 156 | 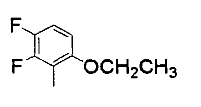 | 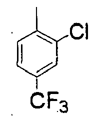 | Amorphous MS(APCI) m/z:498/500[M+H]⁺ |

**Table 20**

| | | | |
|---|---|---|---|
| 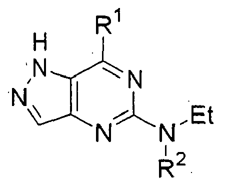 | | | |

| Examples | R¹ | R² | Physical Constants |
|---|---|---|---|
| 157 | 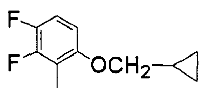 | 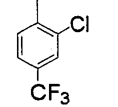 | Amorphous MS(APCI) m/z:524/526[M+H]⁺ |
| 158 | 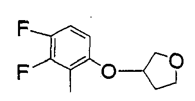 | 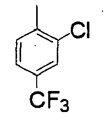 | Amorphous MS(APCI) m/z:540/542[M+H]⁺ |
| 159 | 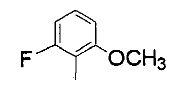 | 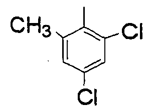 | Amorphous MS(APCI) m/z:446/448[M+H]⁺ |
| 160 | 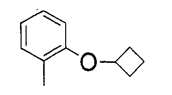 | 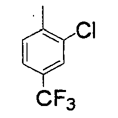 | Amorphous MS(APCI) m/z:488/490[M+H]⁺ |
| 161 | 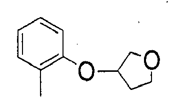 | 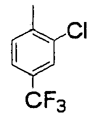 | Amorphous MS(APCI) m/z:504/506[M+H]⁺ |
| 162 | 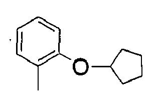 | 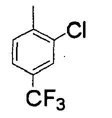 | Amorphous MS(APCI) m/z:502/ 504[M+H]⁺ |
| 163 | 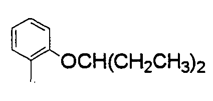 | 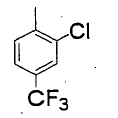 | Amorphous MS(APCI) m/z:504/506[M+H]⁺ |
| 164 | 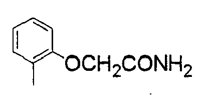 | 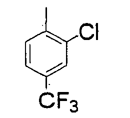 | Amorphous MS(APCI) m/z:491/493[M+H]⁺ |

**Table 21**

| | | | |
|---|---|---|---|
| 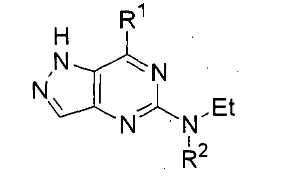 | | | |

| Examples | R¹ | R² | Physical Constants |
|---|---|---|---|
| 165 | 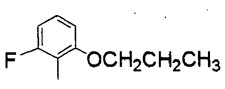 | 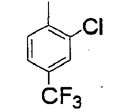 | Amorphous MS(APCI) m/z:494/496[M+H]⁺ |
| 166 | 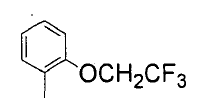 | 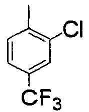 | Amorphous MS(APCI) m/z:516/518[M+H]⁺ |
| 167 | 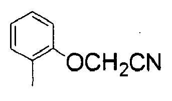 | 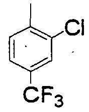 | Amorphous MS(APCI) m/z:473/475[M+H]⁺ |
| 168 | 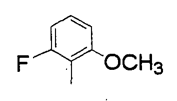 | 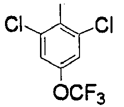 | Amorphous MS(APCI) m/z:516/518[M+H]⁺ |
| 169 | 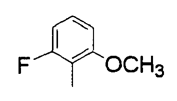 | 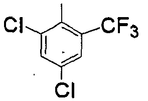 | Amorphous MS(APCI) m/z:500/502[M+H]⁺ |
| 170 | 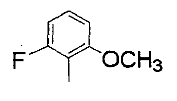 | 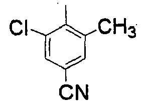 | Amorphous MS(APCI) m/z:437/439[M+H]⁺ |
| 171 | 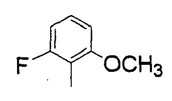 | 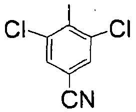 | Amorphous MS(APCI) m/z:457/459[M+H]⁺ |
| 172 | 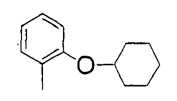 | 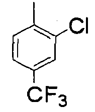 | Amorphous MS(APCI) m/z:516/518[M+H]⁺ |

**Table 22**

| | | | |
|---|---|---|---|
| 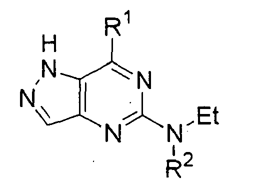 | | | |

| Examples | R¹ | R ² | Physical Constants |
|---|---|---|---|
| 173 | 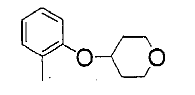 | 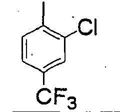 | Amorphous MS(APCI) m/z:518/520[M+H]⁺ |
| 174 | 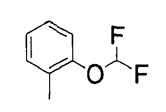 | 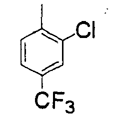 | Amorphous MS(APCI) m/z:484/486[M+H]⁺ |

**Table 23**

| | | | | |
|---|---|---|---|---|
| 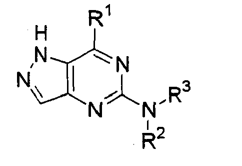 | | | | |

| Examples | R¹ | R² | R³ | Physical Constants |
|---|---|---|---|---|
| 175 | 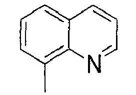 | 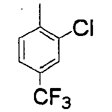 | Me | Amorphous MS (APCI) m/z:455/457[M+H]⁺ |
| 176 | 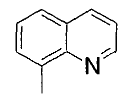 | 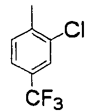 | Pr | Amorphous MS (APCI) m/z:483/485[M+H]⁺ |
| 177 | 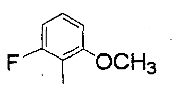 | 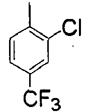 | CH₂CF₃ | Amorphous MS (APCI) m/z:520/522[M+H]⁺ |
| 178 | 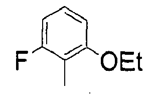 | 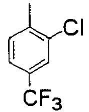 | CH₂CF₃ | Amorphous MS (APCI) m/z:534/536[M+H]⁺ |

### Example 179

### (1)N-[2-Chloro-4-(trifluoromethyl)phenyl]-3-nitro-N-ethyl-7-quinoline-8-yl-1H-pyrazolo[4,3-d]pyrimidine-5-amine

Trifluoromethanesulfonic anhydride(21.3 µL) was added dropwise to tetramethylammonium nitrate(18.1 mg) dissolved in dichloromethane(1.5 mL) and the mixture was stirred at room temperature for 1.5 hours. To the reaction mixture was added N-[2-chloro-4-(trifluoromethyl)pheny]-7-quinoline-8-yl-N-ethyl-1H-pyrazolo[4,3-d]pyrimidine-5-amine(a compound obtained in Example 5) and the mixture was stirred under heating at 80°C for 15 minutes using a microwave reaction apparatus. After being cooled to room temperature, a saturated aqueous solution of sodium bicarbonate was poured into the reaction mixture, and the product was extracted with chloroform. The organic extraction layer was dried over sodium sulfate and the solvent was evaporated under reduced pressure. The resulted residue was purified by using a silica gel column chromatography (hexane:ethyl acetate=1:0 to 1:1) to give the titled compound(38.2 mg, 88% yield) as a yellow amorphous powder. MS(APCI)m/z:514/516[M+H]⁺.

### (2)3-Amino-N-[2-chloro-4-(trifluoromethyl)phenyl]-N-ethyl-7-quinolin-8-yl-1H-pyrazolo[4,3-d]pyrimidine-5-amine

N-[2-Chloro-4-(trifluoromethyl)phenyl]-3-nitro-N-ethyl-7-quinolin-8-yl-1H-pyrazolo[4,3-d]pyrimidine-5-amine(a compound obtained in (1) above) was dissolved in water(0.5 mL) and ethanol(0.5 mL), iron powder(27 mg) and an aqueous 36% HCl solution were added thereto and the mixture was stirred under reflux for 1.5 hours. After being cooled to room temperature, a saturated aqueous solution of sodium bicarbonate was poured into the reaction mixture, and the product was extracted with ethyl acetate. The resulted residue was purified by using a silica gel column chromatography (hexane:ethyl acetate=9:1 to 0:1) to give the titled compound(31.6 mg, 67% yield) as a red amorphous powder. MS (APCI) m/z:484/486[M+H]⁺.

### Example 180

### (1)3-Bromo-N-[2-chloro-4-(trifluoromethyl)phenyl]-N-ethyl-7-quinolin-8-yl-1H-pyrazolo[4,3-d]pyrimidine-5-amine

To a solution of N-[2-chloro-4-(trifluoromethyl)pheny]-N-ethyl-7-quinolin-8-yl-1H-pyrazolo[4,3-d]pyrimidine-5-amine(a compound obtained in Example 5) (267 mg) in N,N-dimethylformamide (1.5 mL) was added N-bromosuccinamide(121.6 mg) and the mixture was stirred at 70°C for 15 hours. After being cooled to room temperature, water was added to the reaction mixture and the product was extracted with ethyl acetate. The organic extraction layer was dried over sodium sulfate and the solvent was evaporated under reduced pressure. The resulted residue was purified by using a silica gel column chromatography (hexane:ethyl acetate=5:1) to give the titled compound(270 mg, 86% yield) as a yellow amorphous powder. MS(APCI)m/z:547/549[M+H]⁺.

### (2)N-[2-Chloro-4-(trifluoromethyl)phenyl]-N-ethyl-3-methyl-7-quinolin-8-yl-1H-pyrazolo[4,3-d]pyrimidine-5-amine

A mixture of 3-Bromo-N-[2-chloro-4-(trifluoromethyl)phenyl]-N-ethyl-7-quinolin-8-yl-1H-pyrazolo[4,3-d]pyrimidine-5-amine(a compound obtained in (1) above)(40 mg), trimethylboroxine(10 µL), 1,1-bis(diphenylphosphino)ferrocene dichloropalladium(II) (3 mg) and pottasium phosphate(47 mg) in 1,4-dioxane(1 mL) was stirred at 120°C(500 W) for 1.5 hours using a microwave reaction apparatus. After being cooled to room temperature, 1,1-bis(diphenylphosphino)ferrocene dichloropalladium(II)(3 mg) and trimethylboroxin(10 µL) were added and the mixture was further stirred at 120°C (500 W) for 1.5 hours using a microwave reaction apparatus. After being cooled to room temperature, a saturated aqueous solution of sodium bicarbonate was added to the reaction mixture and the product was extracted with ethyl acetate. The organic extraction layer was dried over sodium sulfate and the solvent was evaporated under reduced pressure. The resulted residue was purified by using a silica gel column chromatography (hexane:ethyl acetate=1:0 to 2:3) to give the titled compound(24.1 mg, 69% yield) as a brown amorphous powder. MS(APCI)m/z:483/485[M+H]⁺.

### Example 181

### 2-{5-[[2-Chloro-4-(trifluoromethyl)phenyl](ethyl)amino]-1H-pyrazolo[4,3-d]pyrimidine-7-yl}-3-fluorophenol

To N-[2-chloro-4-(trifluoromethyl)phenyl]-N-ethyl-7-(2-fluoro-6-methoxyphenyl)-1H-pyrazolo[4,3-d]pyrimidine-5-amine(a compound obtained in Example 1) (100 mg) dissolved in dichloromethane(2.0 mL), was added 1.0M tribromoborane-dichloromethane solution(0.64 mL) under argon gas atmosphere and the mixture was stirred at room temperature overnight. Water was added to the reaction mixture and the product was extracted with ethyl acetate. The separated organic layer was washed with a saturated aqueous solution of sodium bicarbonate and a saturated brine successively, dried over sodium sulfate and the solvent was evaporated under reduced pressure. The resulted residue was purified by using a silica gel column chromatography (hexane:ethyl acetate=10:1 to 3:1) to give the titled compound(76.8 mg, 79%yield) as a colorless solid. MS(APCI)m/z:434/436[M+H]⁺.

### Example 182

### N-[2-Chloro-4-(trifluoromethyl)phenyl]-N-ethyl-7-(6-hydroxyphenyl)-1H-pyrazolo[4,3-d]pyrimidine-5-amine was obtained by treating N-[2-chloro-4-(trifluoromethyl)phenyl]-N-ethyl-7-(6-methoxyphenyl)-1H-pyrazolo[4,3-d]pyrimidine-5-amine in a similar manner. MS(APCI)m/z:452/454[M+H]⁺.

### Example 183

### (1)N-[2,4-Dichloro-5-(methylsulfonyl)phenyl]-N-ethyl-7-(2-fluoro-6-methoxyphenyl)-1H-pyrazolo[4,3-d]pyrimidine-5-amine

N-[2,4-Dichloro-5-(methylsulfinyl)phenyl]-N-ethyl-7-(2-fluoro-6-methoxyphenyl)-1H-pyrazolo[4,3-d]pyrimidine-5-amine

m-Chloroperbenzoic acid(20 mg) was added to N-[2,4-dichloro-5-(methylthio)phenyl]-N-ethyl-7-(2-fluoro-6-methoxyphenyl)-1H-pyrazolo[4,3-d]pyrimidine-5-amine(38 mg) in dichloromethane(5 mL) and the mixture was stirred at room temperature for three days. m-Chloroperbenzoic acid(8 mg) was further added to the reaction mixture and it was stirred at room temperature for a day. An aqueous solution of sodium sulfite (2 g of sodium sulfite/20 mL of water) was added to the solution and it was stirred at room temperature for three hours. A saturate aqueous solution of sodium bicarbonate was added to the solution and the product was extracted twice with chloroform. The organic extraction layer was dried over sodium sulfate and the solvent was evaporated under reduced pressure. The resulted residue was purified by using a silica gel column chromatography (hexane:ethyl acetate=1:1 to 0:1) to give the compound below.

N-[2,4-Dichloro-5-(methylsulfonyl)phenyl]-N-ethyl-7-(2-fluoro-6-methoxyphenyl)-1H-pyrazolo[4,3-d]pyrimidine-5-amine(17.8 mg, 44% yield) was obtained as a yellow amorphous powder. MS (APCI)m/z:510/512[M+H]⁺.

N-[2,4-Dichloro-5-(methylsulfinyl)phenyl]-N-ethyl-7-(2-fluoro-6-methoxyphenyl)-1H-pyrazolo[4,3-d]pyrimidine-5-amine(22.9 mg, 59% yield) was obtained as a yellow amorphous powder. MS(APCI)m/z:494/496[M+H]⁺.

### Example 184

The next compounds were obtained by treating the corresponding compound in a similar manner to example 183.

N-[2-Chloro-4-trifluoromethylphenyl]-N-ethyl-7-(2-methylsulfonyl-6-methoxyphenyl)-1H-pyrazolo[4,3-d]pyrimidine-5-amine was obtained. MS(APCI)m/z:526/528[M+H]⁺.

N-[2-Chloro-4-trifluoromethylphenyl]-N-ethyl-7-(2-methylsulfinyl-6-methoxyphenyl)-1H-pyrazolo[4,3-d]pyrimidine-5-amine was obtained. MS(APCI)m/z:510/512[M+H]⁺.

### Example 185

### (1)N-[2-Chloro-4-(trifluoromethyl)phenyl]-7-(3,4-dihydro-1,8-naphthyridine-1(2H)-yl)-N-ethyl-2-(4-methoxybenzyl)-2H-pyrazolo[4,3-d]pyrimidine-5-amine

A mixture of 7-chloro-N-[2-chloro-4-trifluoromethylphenyl]-N-ethyl-2-(4-methoxybenzyl)-2H-pyrazolo[4,3-d]pyrimidine-5-amine(a compound obtained in reference example 16)(50 mg), 3,4-dihydro-1,8-naphthyridine(27 mg), tris(dibenzylideneacetone)dipalladium(0)(3.7 mg), 4,5-bis(diphenylphosphino)-9,9-dimethylxanthene(6.9 mg) and cesium carbonate(65 mg) in 1,4-dioxane(1.0 mL) was degassed by passing argon gas under argon atmosphere, and heated under reflux overnight. After being cooled to room temperature, water and ethyl acetate were added to the reaction mixture and it was stirred for 5 minutes. The separated organic layer was washed with a saturated brine, dried over sodium sulfate and the solvent was evaporated under reduced pressure. The resulted residue was purified by using a silica gel column chromatography (hexane:ethyl acetate=5:2 to 4:3) to give the titled compound(42.4 mg, 71% yield) as a colorless solid.
MS(APCI)m/z:594/596[M+H]⁺.

### (2) N-[2-chloro-4-(trifluoromethyl)phenyl]-7-(3,4-dihydro-1,8-naphthyridin-1(2H)-yl)-N-1H-pyrazolo[4,3-d]pyrimidine-5-amine

N-[2-Chloro-4-(trifluoromethyl)phenyl]-7-(3,4-dihydro-1,8-naphthyridin-1(2H)-yl)-N-ethyl-2-(4-methoxybenzyl)-2H-pyrazolo[4,3-d]pyrimidine-5-amine(a compound obtained in (1) above) (39 mg) was dissolved in trifluoroacetic acid(2.0 mL) and heated under reflux overnight. After being cooled to room temperature, the reaction mixture was concentrated under reduced pressure, and a saturated aqueous solution of sodium bicarbonate and ethyl acetate were added thereto. The separated organic layer was washed with a saturated brine, dried over sodium sulfate and the solvent was evaporated under reduced pressure. The resulted residue was purified by using a silica gel column chromatography (hexane:ethyl acetate=5:2 to 20:13) and the crude crystalline was recrystallized from n-haxane-ethyl acetate to give the titled compound(136 mg, 67% yield) as a colorless solid. MS(APCI)m/z:474/476[M+H]⁺.

### Example 186

### (1)N'5'-[2-Chloro-4-(trifluoromethyl)phenyl]-N'5'-ethyl-2-(4-methoxybenzyl)-N'7',N'7-bis(2-methoxyethyl)-2H-pyrazolo[4,3-d]pyrimidine-5,7-amine

A mixture of 7-chloro-N-[2-chloro-4-(trifluoromethyl)phenyl]-N-ethyl-2-(4-methoxybenzyl)-2H-pyrazolo[4,3-d]pyrimidine-5-amine(a compound obtained in Reference Example 16) (75 mg), N,N-diisopropylethylamine(18.2 mg) and bis(2-methoxyethyl)amine(93.7 mg) in N-methylpyrrolidone(1.1 mL) was stirred at outside temperature of 110°C under argon gas atmosphere overnight. After being cooled to room temperature, water and chloroform were added to the reaction mixture, the separated organic layer was washed with a saturated brine, dried over sodium sulfate and the solvent was evaporated under reduced pressure. The resulted residue was purified by using a silica gel column chromatography (hexane:ethyl acetate=10:1 to 5:2) to give the titled compound(84.3 mg) quantitatively as a pale yellow liquid. MS(APCI)m/z:593/595[M+H]⁺. (2)N'5'-[2-Chloro-4-(trifluoromethyl)phenyl]-N'5'-ethyl-N'7',N'7-bis(2-methoxyethyl)-1H-pyrazolo[4,3-d]pyrimidine-5,7-amine

N'5'-[2-Chloro-4-(trifluoromethyl)phenyl]-N'5'-ethyl-2-(4-methoxybenzyl)-N'7',N'7-bis(2-methoxyethyl)-2H-pyrazolo[4,3-d]pyrimidine-5,7-amine(a compound obtained in (1) above) (80 mg) was dissolved in trifluoroacetic acid(1.5 mL) under argon atmosphere and heated under reflux overnight. After being cooled to room temperature, the reaction mixture was concentrated under reduced pressure, and a saturated aqueous solution of sodium bicarbonate and ethyl acetate were added thereto. The separated organic layer was washed with a saturated brine, dried over sodium sulfate and the solvent was evaporated under reduced pressure. The resulted residue was purified by using a silica gel column chromatography (chloroform to chloroform:metanol=20:3) to give the titled compound(46.6 mg, 72% yield) as a yellow viscous oil.
MS(APCI)m/z:473/475[M+H]⁺.

### Examples 187-211

Compounds in the table 24-27 below were obtained by treating the corresponding compound in the similar manner to any of examples.

**Table 24**

| | | | |
|---|---|---|---|
| 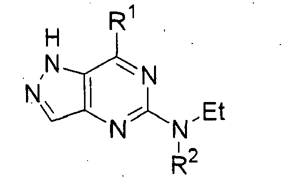 | | | |

| Examples | R¹ | R² | Physical Constants |
|---|---|---|---|
| 187 | -NHCH(C₂H₅)₂ | 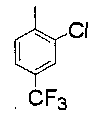 | Amorphous MS(APCI) m/z:427/429[M+H]⁺ |
| 188 | 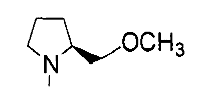 | 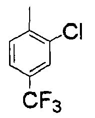 | Amorphous MS(APCI) m/z:455/457[M+H]⁺ |
| 189 | -NHCH(C₃H₇)₂ | 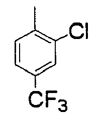 | Amorphous MS(APCI) m/z:455/457[M+H]⁺ |
| 190 | -N(C₃H₇)CH₂CH₂OCH₃ | 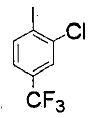 | Amorphous MS(APCI) m/z:457/459[M+H]⁺ |
| 191 | 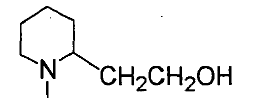 | 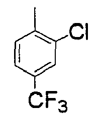 | Amorphous MS(APCI) m/z:469/471[M+H]⁺ |

**Table 25**

| | | | |
|---|---|---|---|
| 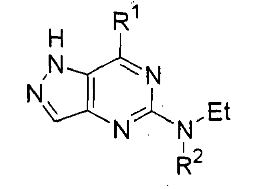 | | | |

| Examples | R¹ | R² | Physical Constants |
|---|---|---|---|
| 192 | -N(CH₂CH₂OCH₃)₂ | 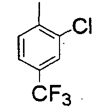 | Amorphous MS(APCI) m/z:473/475[M+H]⁺ |
| 193 | 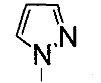 | 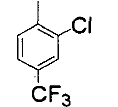 | Amorphous MS(APCI) m/z:408/410[M+H]⁺ |
| 194 | -N(C₂H₅)CH(CH₂OCH₃)₂ | 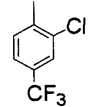 | Amorphous MS(APCI) m/z:487/489[M+H]⁺ |
| 195 | -N(C₂H₅)CH(C₃H₇)₂ | 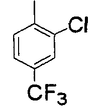 | Amorphous MS(APCI) m/z:483/485[M+H]⁺ |
| 196 | -N(C₂H₅)CH(C₂H₅)₂ | 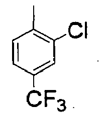 | Amorphous MS(APCI) m/z:455/457[M+H]⁺ |
| 197 | 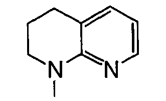 | 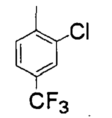 | Amorphous MS(APCI) m/z:474/476[M+H]⁺ |
| 198 | 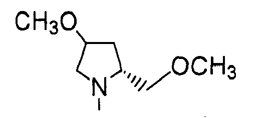 | 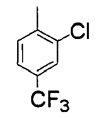 | Amorphous MS(APCI) m/z:485/487[M+H]⁺ |
| 199 | 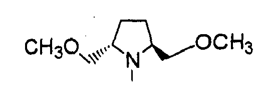 | 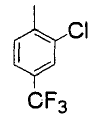 | Amorphous MS(APCI) m/z:499/501[M+H]⁺ |

**Table 26**

| | | | |
|---|---|---|---|
| 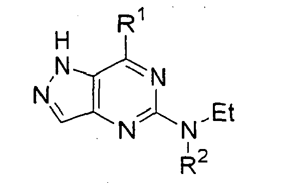 | | | |

| Examples | R¹ | R² | Physical Constants |
|---|---|---|---|
| 200 | 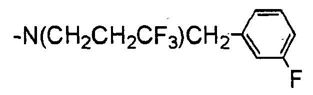 | 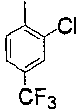 | Amorphous MS(APCI) m/z:561/563[M+H]⁺ |
| 201 | 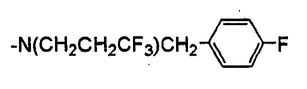 | 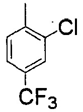 | Amorphous MS(APCI) m/z:561/563[M+H]⁺ |
| 202 | 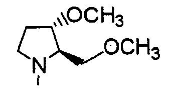 | 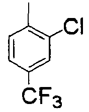 | Amorphous MS(APCI) m/z:485/487[M+H]⁺ |
| 203 | 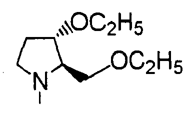 | 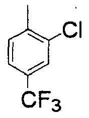 | Amorphous MS(APCI) m/z:513/515[M+H]⁺ |
| 204 | 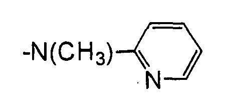 | 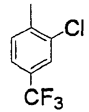 | Amorphous MS(APCI) m/z:448/450[M+H]⁺ |
| 205 | -N(C₄H₉)CH₂CH₂OCH₃ | 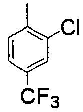 | Amorphous MS(APCI) m/z:471/473[M+H]⁺ |
| 206 | -N(C₄H₉)CH₂CH₂OC₂H₅ | 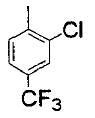 | Amorphous MS(APCI) m/z:485/487[M+H]⁺ |
| 207 | -N(CH₂CH₂OCH₅)CH(C₃H₇)₂ | 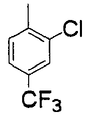 | Amorphous MS(APCI) m/z:513/515[M+H]⁺ |

Table 27

| | | | |
|---|---|---|---|
| 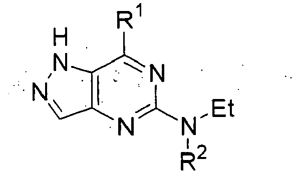 | | | |

| Examples | R¹ | R² | Physical Constants |
|---|---|---|---|
| 208 | -N(C₄H₉)CH(C₃H₇)CH₂OC₂H₅ | 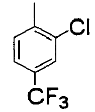 | Amorphous MS(APCI) m/z:513/515[M+H]⁺ |
| 209 | -N(C₄H₉)CH₂CH(C₂H₅)OCH₃ | 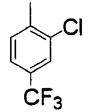 | Amorphous MS(APCI) m/z:499/501[M+H]⁺ |
| 210 | 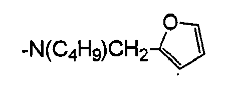 | 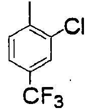 | Amorphous MS(APCI) m/z:493/495[M+H]⁺ |
| 211 | 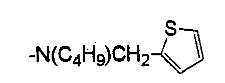 | 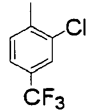 | Amorphous MS(APCI) m/z:509/511[M+H]⁺ |

### Reference Example 1

### Methyl 4-nitro-1H-pyrazole-3-carboxylate

Conc. sulfuric acid(8.2 mL) was added to 4-nitro-1H-pyrazole-3-carboxylic acid(163.8 g) dissolved in methanol(1.64 L) under argon atmosphere and the mixture was heated under reflux for 3.5 hours. After being cooled to room temperature, the reaction mixture was concentrated under reduced pressure and the resulted residue was triturated in diisopropyl ether. The precipitate was collected by filtration to give the titled compound(153.2 g, 86% yield) as a colorless crystalline.
mp.115-117°C, MS(ESI)m/z:170 [M-H]⁻.

### Reference Example 2

### Methyl 1-(4-methoxybenzyl)-4-nitro-1H-pyrazole-3-carboxylate

Sodium hydride(50.02 g) was carefully added to N,N-dimethylformamide(500 mL) at room temperature under argon atmosphere. The mixture was stirred under ice-cooling and methyl 4-nitro-1H-pyrazole-3-carboxylate(178.3 g)(a compound obtained in Reference Example 1) dissolved in N,N-dimethylformamide(880 mL) was added dropwise thereto. Further it was stirred at the same temperature for an hour, 4-methoxybenzylchrolide(159.5 mL) was added dropwise and the stirring was continued at room temperature overnight. To the reaction mixture, were added ice and acetic acid successively, and the product was extracted with ethyl acetate after the pH was adjusted to 7. The organic extraction layer was washed with saturated brine-water(1:1) and then water, dried over sodium sulfate and the solvent was evaporated under reduced pressure. The resulted residue was purified by using a silica gel column chromatography (n-haxane:ethyl actate=10:1 to 3:1) to give the titled compound(210.3 g, 69% yield) and methyl 1-(4-methoxybenzyl)-4-nitro-1H-pyrazole-5-carboxylate(47.3 g, 16% yield) as a colorless oil, respectively.
MS (APCI)m/z:309[M+NH₄]⁺.

### Reference Example 3

### 1-(4-Methoxybenzyl)-4-nitro-1H-pyrazole-3-carboxamide

7N Ammonia/methanol(230 mL) was added at room temperature to methyl 1-(4-methoxybenzyl)-4-nitro-1H-pyrazole-3-carboxylate(37.3 g) (a compound obtained in Reference Example 2) dissolved in methanol(50 mL) and the mixture was stirred overnight. The precipitated crystalline in the solution was washed with diisopropyl ether and collected by filtration to give the titled compound (37.3 g, 83% yield) as a pale yellow crystalline.
mp.192-193°C, MS(ESI)m/z:294[M+NH₄]⁺.

### Reference Example 4

### 4-Amino-1-(4-methoxybenzyl)-1H-pyrazole-3-carboxamide hydrochloride

Iron(31.5 g) and conc.HCl(5.8 mL) were added to a mixture of 1-(4-methoxybenzyl)-4-nitro-1H-pyrazole-3-carboxamide (39.3 g) (a compound obtained in Reference Example 3) and ethanol-water(290 mL - 290 mL), and the mixture was stirred at 80°C for 1.5 hours. The solution was adjusted to pH 8 by adding potassium carbonate and the mixture was filtered through Celite using ethyl acetate. The filtrate was washed with water, dried over sodium sulfate and the solvent was evaporated under reduced pressure. The residue was dissolved in ethyl acetate(230 mL) and the product was changed into powder by adding 4N HCl/ethyl acetate (71 mL). It was recrystalized from diisopropyl ether-methanol to give the titled compound(22 g, 55% yield) as an orange-yellow crystalline.
mp.253°C, MS(ESI)m/z:247[M+H]⁺.

### Reference Example 5

### 2-(4-Methoxybenzyl)-2H-pyrazolo[4,3-d]pyrimidine-5,7(4H,6H)-dione

4-Amino-1-(4-methoxybenzyl)-1H-pyrazole-3-carboxamide hydrochloride(174.5 g)(a compound obtained in Reference Example 4) and urea(136.9 g) were dissolved in ethyleneglycol(1 L) and stirred at 200°C for two hours. After being cooled to room temperature, water was added and the precipitated crystalline was washed with water and collected by filtration to give the titled compound(114 g, 67% yield) as a gray crystalline.
mp.342-344°C, MS(ESI)m/z:290[M+H]⁺.

### Reference Example 6

### 5,7-Dichloro-2-(4-methoxybenzyl)-2H-pyrazolo[4,3-d]pyrimidine

Phosphoryl chloride(300 mL) and N,N-diisopropylethylamine(143.4 mL) were added successively to 2-(4-Methoxybenzyl)-2H-pyrazolo[4,3-d]pyrimidine-5,7 (4H, 6H) -dione (116. 6 g) (a compound obtained in Reference Example 5) dissolved in toluene(200 mL) and the solution was heated under reflux for 1.5 hours. After being cooled to room temperature, the solvent was evaporated under reduced pressure and the residue was azeotropically distilled by adding toluene. It was dissolved again in toluene, washed with cold water and dried over sodium sulfate. The solvent was evaporated under reduced pressure, the product was purified by using a silica gel column chromatography (hexane:ethyl acetate=4:1 to 2:1) and recrystalized from hexane-ethyl acetate to give the titled compound(109.7 g, 83% yield) as a light blue crystalline.
mp.108-109°C, MS(APCI)m/z:309/311[M+H]⁺.

### Reference Example 7

### 5-Chloro-7-(2-fluoro-6-methoxyphenyl)-2-(4-methoxybenzyl)-2H-pyrazolo[4,3-d]pyrimidine

5,7-Dichloro-2-(4-methoxybenzyl)-2H-pyrazolo[4,3-d]pyrimidine (a compound obtained in Reference Example 6)(5.0 g), (2-fluoro-6-methoxyphenyl)boric acid(3.22 g), [1,1'-bis(diphenylphosphino)ferrocene]dichloropalladium(II) dichloromethane complex(0.66 g) and tribasic potassium phosphate(13.7 g) were dissolved in 1,4-dioxane(60 mL) and the flask was degassed after argon gas was passed through the solution and it was stirred at 80°C under argon atmosphere overnight. After being cooled to room temperature, (2-fluoro-6-methoxyphenyl)boric acid(1.24 g) and [1,1'-bis(diphenylphosphino)ferrocene]dichloropalladium(II) dichloromethane complex(0.66 g) were further added and the solution was stirred at 80°C for 7 hours. After being cooled to room temperature, the solution was diluted with ethyl acetate (150 mL) and poured into ice water(200 mL), and the ethyl acetate layer was separated. The separated organic layer was washed with a saturated brine(100 mL), dried over sodium sulfate and the solution was concentrated under reduced pressure. The resulted residue was purified by using a silica gel column chromatography (hexane:ethyl acetate=3:1 to 1:1), the solid product was triturated in hexane-ethyl acetate-diisopropyl ether and the precipitate was collected by filtration to give the titled compound(3.83 g, 59% yield) as a light green amorphous.
MS(APCI)m/z:399/401[M+H]⁺.

### Reference Example 8

### Potassium 2-fluoro-6-methoxyphenyltrifluoroborate

2-Fluoro-6-methoxyphenylboric acid(40 g) was dissolved in methanol(55 mL) and vigorously stirred. 4.5M aq. solution of potassium hydrogen fluoride(183 mL) was added thereto over 20 seconds and the mixture was stirred at room temperature overnight. The precipitates were collected by filtration, washed with water(15 mL) three times, and acetonitrile(20 mL) once. The resulted crystalline was dried to give the titled compound(40.4 g, 74% yield) as a colorless crystalline. MS(APCI)m/z:193[M-K]⁻.

The above washings of water and acetonitrile were combined, acetonitrile was evaporated under reduced pressure and the precipitates were filtered. The crystalline on the filter was washed with acetonitrile and dried to give the titled compound(3.50 g, 16% yield) as a colorless crystalline. MS(APCI)m/z:193[M-K]⁻.

### Reference Example 9

### 5-Chloro-7-(2-fluoro-6-methoxyphenyl)-2-(4-methoxybenzyl)-2H-pyrazolo[4,3-d]pyrimidine

5,7-Dichloro-2-(4-methoxybenzyl)-2H-pyrazolo[4,3-d]pyrimidine(5.00 g) (a compound obtained in Reference Example 6), potassium 2-fluoro-6-methoxyphenyltrifluoroborate(4.51 g)(a compound obtained in Reference Example 8), N,N-diisopropylethylamine(6.28 g), [1,1'-bis(diphenylphosphine)ferrocene]dichloropalladium(II)dichlo romethane complex(0.661 g) dissolved in tetrahydrofuran(160 mL) and water(32 mL) under argon atmosphere were heated under reflux for 1.5 hours. After being cooled to room temperature, tetrahydrofuran was evaporated under reduced pressure and ethyl acetate was added. The separated organic layer was washed with a saturated brine, dried over sodium sulfate and the solvent was evaporated under reduced pressure. The resulting residue was purified by using a NH silica gel column chromatography (ethyl acetate) and the solid product was triturated in diethyl ether- ethyl acetate(5:1), the precipitates were collected by filtration to give the titled compound(5.14 g, 80% yield) as a light red crystalline. mp.180-181°C, MS(APCI)m/z:39.9/401[M+H]⁺.

### Reference Example 10

### N-[2-Chloro-4-(trifluoromethyl)phenyl]-7-(2-fluoro-6-methoxyphenyl)-2-(4-methoxybenzyl)-2H-pyrazolo[4,3-d]pyrimidine-5-amine

5-Chloro-7-(2-fluoro-6-methoxyphenyl)-2-(4-methoxybenzyl)-2H-pyrazolo[4,3-d]pyrimidine(9.06 g)(a compound obtained in Reference Example 9), 2-chloro-4-trifluoromethylaniline(6.22 g), tris(dibenzylidenecetone)dipalladium(0)(0.832 g), 4,5-bis(diphenylphosphino)-9,9-dimethylxantene(1.58 g) and cesium carbonate(14.8 g) were dissolved in 1,4-dioxane(250 mL) under argon atmosphere, the solution was degassed by passing argon gas and it was heated under reflux overnight. After being cooled to room temperature, water(300 mL) and ethyl acetate(300 mL) were added to the solution and the mixture was stirred for five minutes. The separated organic layer was washed with a saturated brine, dried over sodium sulfate and the solvent was evaporated under reduced pressure. The resulting residue was purified by using a silica gel column chromatography (hexane:ethyl acetate=4:1) and the solid product was triturated in ethyl acetate-diethyl ether. The precipitates were collected by filtration to give the titled compound(6.61 g, 52% yield) as a pale yellow amorphous. MS(APCI)m/z:558/560[M+H]⁺.

### Reference Example 11

### N-[2-Chloro-4-(trifluoromethyl)phenyl]-N-ethyl-7-(2-fluoro-6-methoxyphenyl)-2-(4-methoxybenzyl)-2H-pyrazolo[4,3-d]pyrimidine-5-amine

N-[2-Chloro-4-(trifluoromethyl)phenyl]-7-(2-fluoro-6-methoxyphenyl)-2-(4-methoxybenzyl)-2H-pyrazolo[4,3-d]pyrimidine-5-amine(8.12 g)(a compound obtained in Reference Example 10) was dissolved in N,N-dimethylformamide(100 mL) and the solution was degassed by passing argon gas. The solution was cooled in ice-acetone bath and iodoethane(3.41 g) and sodium hydride(0.873 g) was added in this order and the mixture was stirred for two minutes under argon atmosphere. The ice-acetone bath was removed and the solution was stirred for one hour. The reaction mixture was poured into an ice-cooled saturated solution of ammonium chloride(200 mL) and the product was extracted with a mixed solvent of ethyl acetate-diethyl ether(300 mL-100 mL). The extracted layer was washed with a saturated brine, dried over sodium sulfate, and the solvent was evaporated under reduced pressure. The resulting residue was purified by using a silica gel column chromatography (hexane:ethyl acetate=3:1 to 11:9) to give the titled product(6.96 g, 82% yield) as an amorphous.
MS(APCI)m/z:586/588[M+H]⁺.

### Reference Example 12

### 7-(Benzyloxy)-5-chloro-2-(4-methoxybenzyl)-2H-pyrazolo[4,3-d]pyrimidine

5,7-Dichloro-2-(4-methoxybenzyl)-2H-pyrazolo[4,3-d]pyrimidine(a compound obtained in Reference Example 6) (44.8 g) was dissolved in tetrahydrofuran(450 mL) under argon atmosphere and benzyl alcohol(16.45 g) was added thereto at room temperature. The reaction mixture was cooled to -78°C and the mixture was stirred at the same temperature for 30 minutes after sodium hydride(60% in oil; 6.08 g) was added by portions. The mixture was warmed up to room temperature and stirred for two days. Water(500 mL) was added to the reaction mixture and extracted with ethyl acetate (1.5 L). The extracted organic layer was washed with a saturated brine, dried over sodium sulfate, and the solvent was evaporated under reduced pressure. The resulting crude crystalline was triturated in isopropyl ether and the precipitate was collected by filtration to give the titled compound(50.6 g, 92 % yield) as a colorless crystalline. mp.149.5-153°C, MS(APCI)m/z:381/383[M+H]⁺.

### Reference Example 13

### 7-Benzyloxy-N-[2-chloro-4-(trifluoromethyl)phenyl]-2-(4-methoxybenzyl)-2H-pyrazolo[4,3-d]pyrimidine-5-amine

A mixture of 7-(Benzyloxy)-5-chloro-2-(4-methoxybenzyl)-2H-pyrazolo[4,3-d]pyrimidine(53.0 g), 2-chloro-4-(trifluoromethyl)aniline(38.1 g), tris(dibenzylideneacetone)dipalladium(0)(5.10 g), 4,5-bis(diphenylphosphino)-9,9-dimethylxanthene(9.66 g)(a compound obtained in Reference Example 12), cesium carbonate(90.7 g) and 1,4-dioxane(1.05 L) was heated under reflux for 16 hours under argon atmosphere. After being cooled to room temperature, ethyl acetate(1 L) was added to the reaction mixture and filtered through Celite. The residue on the Celite was washed with ethyl acetate(1 L) and chloroform(500 mL), the washings were combined with the filtrate, and concentrated under reduced pressure. The resulting residue was crystallized and triturated in ethyl acetate-diethyl ether(1:1) and the crystalline was filtered and washed with ethyl acetate-diethyl ether(1:1). The obtained crystalline was dried to give the titled compound(43.7 g, 58% yield) as a pale yellow crystalline. mp.189-192°C, MS(APCI)M/z:540/S42[M+H]⁺.

### Reference Example 14

### 7-Benzyloxy-N-[2-chloro-4-(trifluoromethyl)phenyl]-N-ethyl-2-(4-methoxybenzyl)-2H-pyrazolo[4,3-d]pyrimidine-5-amine

A suspension of 7-Benzyloxy-N-[2-chloro-4-(trifluoromethyl)phenyl]-2-(4-methoxybenzyl)-2H-pyrazolo[4,3-d]pyrimidine-5-amine(45.4 g) (a compound obtained in Reference Example 13) in tetrahydrofuran(44 mL) and N,N-dimethylformamide(440 mL) was stirred under argon atmosphere, sodium hydrate(60% in oil; 5.05 g) was added in small portions under ice-cooling and the mixture was stirred at room temperature for 30 minutes. Into the reaction mixture cooled in ice again, was added iodoethane(26.2 g) dropwise and it was stirred at room temperature for 2 hours. Into ethyl acetate (1 L) and ice water(500 mL) stirred, was poured the reaction mixture slowly, the organic layer was separated and the aqueous layer was further extracted with ethyl acetate. The combined organic layer was washed with a saturated brine, dried over sodium sulfate and filtered through Celite. The residue on the Celite was washed with ethyl acetate and the combined filtrate was concentrated under reduced pressure. The resulting residue was dissolved in diethyl ether(1 L), washed with water and a saturated brine and dried over sodium sulfate. The solution was filtered through Celite, the residue on the Celite was washed with diethyl ether, and the combined filtrate was concentrated under reduced pressure to give the titled compound(46.7 g, quantitatively) as an yellow amorphous. MS(APCI)m/z:568/570[M+H]⁺.

### Reference Example 15

### 5-N-[2-Chloro-4-(trifluoromethyl)phenyl]ethylamino-2-(4-methoxybenzyl)-2H-pyrazolo[4,3-d]pyrimidine-7-ol

7-Benzyloxy-N-[2-chloro-4-(trifluoromethyl)phenyl]-N-ethyl-2-(4-methoxybenzyl)-2H-pyrazolo[4,3-d]pyrimidine-5-amine (46.7 g) (a compound obtained in Reference Example 14) was dissolved in 4N HCl/1,4-dioxane under ice-cooling and heated under reflux for 19 hours. After being cooled to room temperature, precipitated crystalline was filtered and washed with diethyl ether. It was dried to give 5-N-[2-chloro-4-(trifluoromethyl)phenyl]ethylamino-2-(4-methoxybenzyl)-2H-pyrazolo[4,3-d]pyrimidine-7-ol(14.8 g, 37% yield) as a colorless crystalline.
MS(APCI)m/z:478/480[M+H]⁺.

The mother liquid was concentrated, ethyl acetate(1 L) and water(300 mL) were added to the resulting residue and the mixture was neutralized by adding a saturated aqueous solution of sodium bicarbonate. The separated organic layer was washed with a saturated brine, dried over sodium sulfate and the solvent was evaporated under reduced pressure. The resulting oily residue was purified by using a silica gel column chromatography(hexane:ethyl acetate=4:1 to 1:3) and stirred in diethyl ether to become a crystalline. The obtained crystalline was collected by filtration and dried to give the titled product (17.0 g, 42.7% yield) as a colorless crystalline.
MS(APCI) m/z:478/480[M+H]⁺.

### Reference Example 16

### 7-Chloro-N-[2-chloro-4-(trifluoromethyl)phenyl]-N-ethyl-2-(4-methoxybenzyl)-2H-pyrazolo[4,3-d]pyrimidine-5-amine

5-N-[2-Chloro-4-(trifluoromethyl)phenyl]ethylamino-2-(4-methoxybenzyl)-2H-pyrazolo[4,3-d]pyrimidine-7-ol(31.8 g)(a compound obtained in Reference Example 15) was suspended in phosphorus oxychloride(427 g) and stirred at 60°C for 2.5 hours. After being cooled to room temperature, the solution was concentrated under reduced pressure and the residue was poured into a stirred mixture of ethyl acetate-a 10% aqueous solution of sodium bicarbonate and neutralized by adding a 10% aqueous solution of sodium bicarbonate. The separated organic layer was washed with a saturated brine, dried over sodium sulfate and the solvent was evaporated under reduced pressure. The resulting residue was purified by using a silica gel column chromatography(hexane:ethyl acetate=2:1 to 1:4) and crystallized and triturated in hexane. The obtained crystalline was collected by filtration, washed by hexane, and dried to give the titled product(19.1 g, 58% yield) as a colorless crystalline. MS(APCI)m/z:496/498[M+H]⁺.

### Reference Example 17

### N-[2-Chloro-4-(trifluoromethyl)phenyl]-N-ethyl-7-(2-fluoro-6-metnoxyphenyl)-2-(4-methoxybenzyl)-2H-pyrazolo[4,3-d]pyrimidine-5-amine

7-Chloro-N-[2-chloro-4-(trifluoromethyl)phenyl]-N-ethyl-2-(4-methoxybenzyl)-2H-pyrazolo[4,3-d]pyrimidine-5-amine(50 mg) (a compound obtained in Reference Example 16), potassium 2-fluoro-6-methoxyphenyl-trifluoroborate(29 mg)(a compound obtained in Reference Example 8), N,N-diisopropylethylamine(90µL) and [1,1'-bis(diphenylphosphino)ferrocene]dichloropalladium(II)dichlo romethane complex(7 mg) dissolved in a mixture of tetrahydrofuran(1 mL) and water(0.2 mL) was heated under reflux overnight. After being cooled to room temperature, water and ethyl actetate were added and the mixture was stirred vigorously. The organic layer was separated and the solvent was evaporated under reduced pressure. The resulting residue was purified by using a silica gel column chromatography(hexane:ethyl acetate=85:15 to 65:35) to give the titled compound(57 mg, 97% yield) as an amorphous.
MS(APCI)M/z:586/588[M+H]⁺.

### Reference Example 18

### N-[2-Chloro-4-(trifluoromethyl)phenyl]-N-ethyl-7-(2-fluoro-6-methoxyphenyl)-2-(4-methoxybenzyl)-2H-pyrazolo[4,3-d]pyrimidine-5-amine

7-Chloro-N-[2-chloro-4-(trifluoromethyl)phenyl]-N-ethyl-2-(4-methoxybenzyl)-2H-pyrazolo[4,3-d]pyrimidine-5-amine(4.88 g)(a compound obtained in Reference Example 16), 2-fluoro-6-methoxyphenylboric acid(2.57 g), 2M aq.solution of sodium carbonate(40 mL) and bis(triphenylphosphine)dichloropalladium(II)(707 mg) were dissolved in 1,2-dimethoxyethane(135 mL) under argon atmosphere and it was heated under reflux overnight. After being cooled to room temperature, water and ethyl acetate were added and the mixture was vigorously stirred. The organic layer was separated and the solvent was evaporated under reduced pressure. The resulting residue was purified by using a silica gel column chromatography(hexane:ethyl acetate=90:10 to 65:35) to give the titled compound(1.38 g, 24% yield) as an amorphous solid.
MS(APCI)M/z:586/588[M+H]⁺.

### Reference Example 19

### Methyl 1-(4-methoxybenzyl)-4-nitro-1H-pyrazol-5-carboxamide

7N-Ammonia/methanol(230 mL) was added to methyl 1-(4-methoxybenzyl)-4-nitro-1H-pyrazol-5-carboxylate(47.3 g) (a compound obtained in Reference Example 2) dissolved in methanol(50 mL) and stirred overnight. Crystalline separated out in the solution was washed with diisopropyl ether and collected by filtration to give the titled compound(39.0 g, 87% yield) as a yellow crystalline.
MS(APCI)m/z:294[M+NH₄]⁺.

### Reference Example 20

### 4-Amino-1-(4-methoxybenzyl)-1H-pyrazol-5-carboxamide hydrochloride

Iron(31.5 g) and conc.HCl(5.8 mL) were added to methyl 1-(4-methoxybenzyl)-4-nitro-1H-pyrazol-5-carboxamide(39.0 g) (a compound obtained in Reference Example 19) dissolved in a mixture of ethanol and water (290 mL - 290 mL) and it was stirred at 80°C for 1.5 hours. The solution was adjusted to pH 8 by adding potassium carbonate and filtered through Celite using ethyl acetate. The filtrate was washed with water, dried over sodium sulfate and the solvent was evaporated under reduced pressure. The residue was dissolved in ethyl acetate(230 mL) and a crude crystalline was obtained by adding 4N HCl/ethyl acetate(71 mL). It was recrystalized from diisopropanol-methanol to give the titled compound(22.0 g, 55% yield) as an orange yellow crystalline.
mp.196-197°C, MS(ESI)m/z:247[M+H]⁺(a free form).

### Reference Example 21

### 1-(4-methoxybenzyl)-1H-pyrazolo[4,3-d]pyrimidine-5,7-diol

4-Amino-1-(4-methoxybenzyl)-1H-pyrazole-5-carboxamide hydrochloride (30.7 7 g)(a compound obtained in Reference Example 20) and urea(29.6 g) dissolved in ethyleneglycol(230 mL) was stirred at 200°C for 2 hours. After being cooled to room temperature, water(1.5 L) was added and the solution was left to stand. The crystalline separated out was washed with water and collected by filtration to give the titled compound (20.08 g, 68% yield) as a gray crystalline. mp.288-292°C, MS(ESI)m/z:273[M+H]⁺.

### Reference Example 22

### 5,7-Dichloro-1-(4-methoxybenzyl)-1H-pyrazolo[4,3-d]pyrimidine

Phosphorus oxychloride(65.9 mL)and N,N-diisopropylamine(23.7 mL) were added successively to 1-(4-methoxybenzyl)-1H-pyrazolo[4,3-d]pyrimidine-5,7-diol(20.0 g)(a compound obtained in Reference Example 21) dissolved in toluene(50 mL) at room temperature and the mixture was heated under reflux for 1.5 hours. After being cooled to room temperature, the solvent was evaporated under reduced pressure and the resulting residue was azeotropically distilled after adding toluene. The residue was dissolved again in toluene, washed with cold water and dried over sodium sulfate. The solvent was evaporated under reduced pressure and the residue was purified by using a silica gel column chromatography(hexane:ethyl acetate=4:1 to 2:1),recrystalized from hexane-ethyl acetate to give the titled compound(19.10 g, 87% yield) as a pale yellow crystalline.
mp.100-101°C, MS (APCI)m/z:305/307[M-Cl+OMe+H]⁺

### Reference Example 23

### 5-Chloro-7-(2-fluoro-6-methoxyphenyl)-1-(4-methoxybenzyl)-1H-pyrazolo[4,3-d]pyrimidine

5,7-Dichloro-2-(4-methoxybenzyl)-1H-pyrazolo[4,3-d]pyrimidine(4.65 g)(a compound obtained in Reference Example 22), potassium 2-fluoro-6-methoxyphenyl-trifluoroborate(3.84 g) (a compound obtained in Reference Example 8), N,N-diisopropylethylamine(5.83 g), [1,1'-bis(diphenylphosphino)ferrocene]dichloropalladium(II)dichlo romethane complex (1 .23 g) dissolved in tetrahydrofuran (150 mL) and water(30 mL) under argon gas atmosphere was heated under reflux for an hour. After being cooled to room temperature, tetrahydrofuran was evaporated under reduced pressure, and ethyl acetate was added. The separated organic layer was washed with a saturated brine, dried over sodium sulfate and the solvent was evaporated under reduced pressure. The resulting residue was purified by using a NH silica gel column chromatography(ethyl acetate), the obtained solid product was triturated in diethyl ether-ethyl acetate(5:1)and the precipitate was collected by filtration to give the titled compound(5.6 g, 93% yield) as a colorless solid.
MS(APCI)m/z:399/401[M+H]⁺.

### Reference Example 24

### N-[2-Chloro-4-(trifluoromethyl)phenyl]-7-(2-fluoro-6-methoxyphenyl)-1-(4-methoxybenzyl)-1H-pyrazolo[4,3-d]pyrimidine-5-amine

5-Chloro-7-(2-fluoro-6-methoxyphenyl)-1-(4-methoxybenzyl)-1H-pyrazolo[4,3-d]pyrimidine(1.0 g)(a compound obtained in Reference Example 23), 2-chloro-4-trifluoromethylaniline(982 mg), tris(dibenzylideneacetone)dipalladium(0)(92 mg), 4,5-bis(diphenylphosphino)-9,9-dimethylxanthene(0.17 g) and cesium carbonate(1.63 g) were mixed in 1,4-dioxane(28 mL), the reaction vessel was degassed after argon gas was passed through the solution and it was heated under reflux under argon atmosphere overnight. After being cooled to room temperature, water(30 mL) and ethyl acetate(30 mL) were added to the reaction mixture, and it was stirred for 5 minutes. The separated organic layer was washed with a saturated brine, dried over sodium sulfate, and the solvent was evaporated under reduced pressure. The resulting residue was purified by using a silica gel column chromatography(hexane:ethyl acetate=4:1) to give the titled compound(1.24 g, 88% yield) as a colorless amorphous.
MS(APCI)m/z:558/560[M+H]⁺.

### Reference Example 25

### N-[2-Chloro-4-(trifluoromethyl)phenyl]-N-ethyl-7-(2-fluoro-6-methoxyphenyl)-1-(4-methoxybenzyl)-1H-pyrazolo[4,3-d]pyrimidine-5-amine

N-[2-Chloro-4-(trifluoromethyl)phenyl]-7-(2-fluoro-6-methoxyphenyl)-1-(4-methoxybenzyl)-1H-pyrazolo[4,3-d]pyrimidine-5-amine(0.86 g)(a compound obtained in Reference Example 24) was dissolved in N,N-dimethylformamide(9.0 mL) and the reaction vessel was degassed after argon gas was passed through the solution. The vessel was cooled in an ice-acetone bath, iodoethane (0.19 mL) and sodium hydride(0.092 g) were added successively thereto and the mixture was stirred for 2 minutes. The ice-acetone bath was removed and the mixture was stirred for 0.5 hours. Under ice-cooling, the reaction mixture was poured into a saturated ammonium chloride solution(20 mL) and the product was extracted with a mixed solvent(ethyl acetate 30 mL; diethyl ether 10 mL). The separated organic layer was washed with a saturated brine, dried over sodium sulfate, and the solvent was evaporated under reduced pressure. The resulting residue was purified by using a silica gel column chromatography(hexane:ethyl acetate=3:1 to 11:9) to give the titled compound(846 mg, 96% yield) as an amorphous. MS(APCI)M/Z:586/588[M+H]⁺.

### Reference Example 26

### N-[2-Chloro-4-(trifluoromethyl)phenyl]-N-ethyl-7-(2-fluoro-6-methoxyphenyl)-1H-pyrazolo[4,3-d]pyrimidine-5-amine

N-[2-Chloro-4-(trifluoromethyl)phenyl]-N-ethyl-7-(2-fluoro-6-methoxyphenyl)-1-(4-methoxybenzyl)-1H-pyrazolo[4,3-d]pyrimidine-5-amine(166 mg)(a compound obtained in Reference Example 25) was dissolved in trifluoroacetic acid(3.0 mL) under argon atmosphere and heated to reflux overnight. After being cooled to room temperature, the reaction mixture was concentrated and a saturated aqueous solution of sodium bicarbonate and ethyl acetate were added. The separated organic layer was washed with a saturated brine, dried over sodium sulfate, and the solvent was evaporated under reduced pressure. The resulting residue was purified by using a silica gel column chromatography(hexane:ethyl acetate=3:1 1 to 3:2). The obtained crude crystalline was recrystalized from hexane-ethyl acetate to give the titled compound(136 mg) quantitatively as a pale yellow crystalline.
MS(APCI)m/z:466/468[M+H]⁺.

### Industrial Applicability

The objective compound [I] of the present invention or a pharmaceutically acceptable salt thereof shows an antagonistic activity against CRF receptors.

Accordingly, the objective compound [I] of the present invention or a pharmaceutically acceptable salt thereof may be used as a agent for the treatment or prevention of depression, anxiety disorder, irritable bowel syndrome(IBS) and the like. Moreover, the objective compound [I] of the present invention is less toxic and has the feature as a safe drug.

## Claims

1. A pyrazolopyrimidine derivative of the generic formula [I] 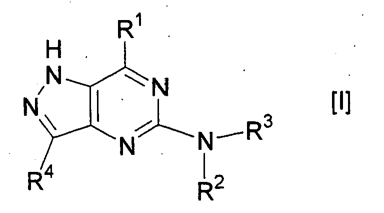 wherein R¹ is an optionally substituted aromatic ring group, an optionally substituted lower alkyl group or an optionally substituted amino group; R² is an optionally substituted aromatic ring group; R³ is an optionally substituted lower alkyl group; and R⁴ is a hydrogen atom, a lower alkyl group, a halogen atom, a nitro group or an amino group;
or a pharmaceutically acceptable salt thereof.

2. A compound of claim 1, wherein R¹ is an optionally substituted aromatic ring group; R² is an optionally substituted aromatic ring group; R³ is a lower alkyl group; and R⁴ is a hydrogen atom.

3. A compound of claim 1, wherein R¹ is an aromatic ring group optionally substituted with 1-5 group(s) selected from a lower alkoxy group optionally substituted with 1-5 halogen atom(s), a halogen atom, an optionally substituted amino group and an optionally substituted alkyl group; R² is an aromatic ring group substituted with 1-5 group(s) selected from a halogen atom, a lower alkoxy group, a lower alkyl group optionally substituted with 1-5 halogen atom(s),an optionally substituted carbamoyl group and an optionally substituted amino group; R³ is a lower alkyl group; and R⁴ is a hydrogen atom.

4. A compound of claim 1, wherein R¹ is an aromatic ring group optionally substituted with 1-5 group(s) selected from a lower alkoxy group optionally substituted with 1-5 halogen atom(s) and a halogen atom; R² is an aromatic ring group substituted with 1-5 group(s) selected from a halogen atom, a lower alkoxy group and a lower alkyl group optionally substituted with 1-5 halogen atom(s); R³ is a lower alkyl group; and R⁴ is a hydrogen atom.

5. A compound of claim 4, wherein R¹ is a phenyl group or quinolyl group optionally substituted with 1-5 group(s) selected from a lower alkoxy group optionally substituted with 1-5 halogen atom(s) and a halogen atom; and R² is a phenyl group substituted with 1-5 group(s) selected from a halogen atom, a lower alkoxy group and a lower alkyl group optionally substituted with 1-5 halogen atom(s) .

6. A compound selected from;
N-[2-Chloro-4-(trifluoromethyl)phenyl]-N-ethyl-7-(2-methoxy-6-fluorophenyl)-1H-pyrazolo[4,3-d]pyrimidine-5-amine,
N-[2-Chloro-4-(trifluoromethyl)phenyl]-N-ethyl-7-quinoline-8-yl-1H-pyrazolo[4,3-d]pyrim-idine-5-amine,
N-(2-Chloro-4-methoxyphenyl)-N-ethyl-7-(2-methoxyphenyl)-1H-pyrazolo[4,3-d]pyrimidine-5-amine
N-[2-Chloro-4-(trifluoromethyl)phenyl]-N-ethyl-7-[2-(trifluoromethoxy)phenyl]-1H-pyrazolo[4,3-d]pyrimidine-5-amine
N-[2-Chloro-4-(trifluoromethyl)phenyl]-N-ethyl-7-(2-ethoxy-6-fluorophenyl)-1H-pyrazolo[4,3-d]pyrimidine-5-amine
N-[2-Chloro-4-(trifluoromethyl)phenyl]-7-[2-(difluoromethoxy)phenyl]-N-ethyl-1H-pyrazolo[4,3-d]pyrimidine-5-amine
N-[2-Chloro-4-(trifluoromethyl)phenyl]-7-(2,3-difluoro-6-methoxyphenyl)-N-ethyl-1H-pyrazolo[4,3-d]pyrimidine-5-amine
N-[2-Chloro-4-(trifluoromethyl)phenyl]-7-[2-(cyclopropylmethoxy)phenyl]-N-ethyl-1H-pyrazolo[4,3-d]pyrimidine-5-amine, and
N-(2-Chloro-4-methoxyphenyl)-N-ethyl-7-(2-fluoro-6-metyhoxyphenyl)-1H-pyrazolo[4,3-d]pyrimidine-5-amine,
or a pharmaceutically acceptable salt thereof.

7. A compound of the formula [II], [III], [V], [VII], [X], [XI], [XII] or the generic formula [XIII], 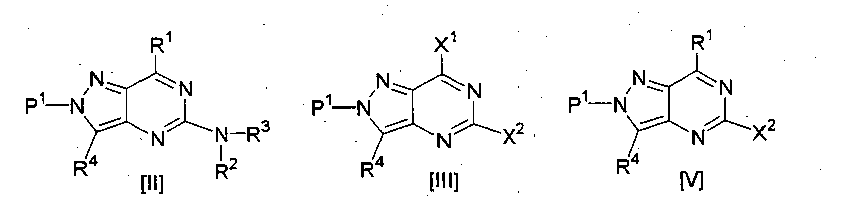 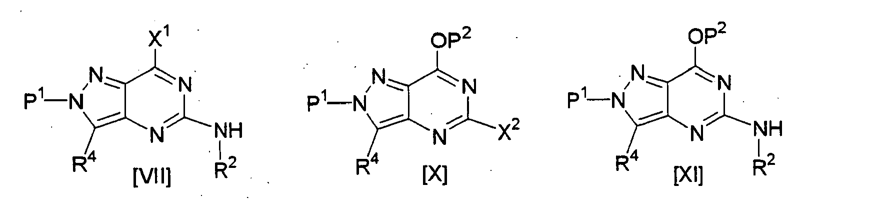 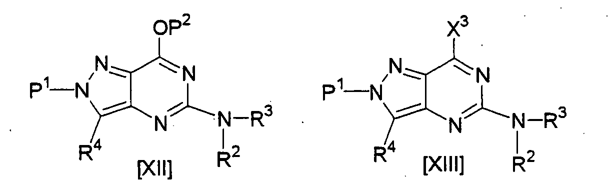 wherein R¹ is an optionally substituted aromatic ring group, an optionally substituted lower alkyl group or an optionally substituted amino group; R² is an optionally substituted aromatic ring group; R³ is an optionally substituted lower alkyl group; and R⁴ is a hydrogen atom, a lower alkyl group, a halogen atom, a nitro group or an amino group; P¹ and P² are protecting groups and X¹, X² and X³ are leaving groups;
or a salt thereof.

8. A compound selected from;
Methyl 1-(4-methoxybenzyl)-4-nitro-1H-pyrazole-3-carboxylate
1-(4-Methoxybenzyl)-4-nitor-1H-pyrazole-3-carboxamide,
4-Amino-1-(4-methoxybenzyl)-1H-pyrazole-3-carboxamide,
2-(4-Methoxybenzyl)-2H-pyrazolo[4,3-d]pyrimidine-5,7(4H,6H)-dione, and
5,7-Dichloro-2-(4-methoxybenzyl)-2H-pyrazolo[4,3-d]pyrimidine;
or a salt thereof.
